# EUROPEAN PATENT APPLICATION

(11) **EP 3 653 656 A1**
(43) Date of publication of application: **20.05.2020**
(21) Application number: 18382814.4
(22) Date of filing: 16.11.2018
(51) Int. Cl.: C08F 220/22, C08F 220/20, A61P 9/00, A61K 31/78, C08F 20/30

(54) **POLYMER FOR LIQUID EMBOLIC AGENTS AND METHOD OF OBTAINING SAME**

(71) Applicant: LVD Biotech S.L., 08620 Sant Vicenç dels Horts (ES)
(72) Inventor: Duocastella Codina, Lluís, 08620 Sant Vicenç dels Horts (ES); Molina Crisol, María, 08620 Sant Vicenç dels Horts (ES); Gómez Castel, Alex, 08620 Sant Vicenç dels Horts (ES); Molins Gutiérrez, Gemma, 08620 Sant Vicenç dels Horts (ES); Palao Suay, Raquel, 28006 Madrid (ES); Aguilar de Armas, María Rosa, 28006 Madrid (ES); Román del Barrio, Julio San, 28006 Madrid (ES)
(74) Representative: Isern Patentes y Marcas S.L.

(57) **Abstract**

The present invention has as an object a polymer of formula (I): 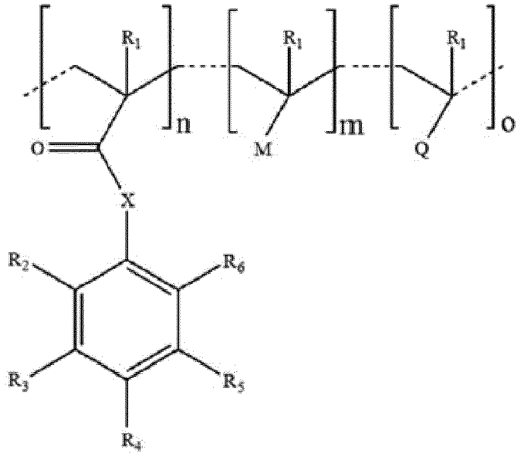 as well as liquid embolic agents containing it.

## Description

### FIELD OF THE INVENTION

The present invention relates to a novel radiopaque liquid embolic agent *per se* and a method for producing same. In particular, it relates to a precipitating liquid embolic agent containing a polymer (homopolymer, copolymer or terpolymer) in a water-miscible organic solvent, which preferably is physiologically acceptable, and more preferably dimethyl sulfoxide (DMSO), as a vehicle in which the product has excellent chemical and mechanical properties for the correct embolization of the region to be devascularized.

### BACKGROUND

Arterial or venous embolization plays a fundamental role in the treatment of a number of vascular diseases. Some of the most common indications include tumor embolization, embolization of arteriovenous malformations and hemorrhages which compromise the hemodynamic stability of the patient or which entail constant transfusion requirements.

This has driven the development of new techniques for the treatment of said diseases, whereby an increasingly higher cure rate and lower rate of adverse effects can be obtained. Among the techniques that have been developed endovascular surgery stands out, whereby complete occlusion of the vessels forming the disease is sought for a curative or adjuvant purpose with respect to microsurgery or radiosurgery.

A range of different products can be used for the embolization of these vascular malformations, such products including *coils* made of metal, hybrid *coils* made of polymers and metal, microparticles, foams and liquids.

Some of the most commonly used embolic agents include liquid embolic agents such as tissue adhesives, such as cyanoacrylates. Cyanoacrylates are low viscosity liquids at room temperature. When they are exposed to an ionic environment (e.g. physiological saline or blood), polymerization is quickly initiated. For that reason, only non-ionic solutions should be used for the preparation and administration of the adhesive. Exposure to insignificant amounts of ionic substances can initiate the polymerization reaction, giving rise to a complete and irreversible blockage of the delivery catheters. In turn, cyanoacrylates cause an inflammatory reaction and deformation of the vessel at the site where polymerization takes place. Although it is considered a permanent agent, part of the embolized vessel can lead to recanalization after several weeks.

Given that cyanoacrylates are radiolucent, they need to be mixed with a radiopaque substance to make them visible. A liposoluble ionic contrast agent obtained from poppy seeds called Lipiodol is most commonly used for this purpose. The amount of Lipiodol used for the mixture with the adhesive influences polymerization time, such that the higher the amount of Lipiodol used, the longer the polymerization time (the amount of Lipiodol used also influences penetration of the embolic agent and, naturally, its radiopacity, the higher the amount of lipiodol, the greater the penetration and the greater the radiopacity). Typically a ratio of between 1:1 and 6:1 of Lipiodol to cyanoacrylate is used, depending on how fast polymerization is to be. Small amounts of adhesive are normally needed, since the substance attracts elements from the blood into it as it polymerizes, giving rise to a significantly higher amount of embolic product than what is expected with said amount of adhesive. The injection techniques are of vital importance when cyanoacrylates are used. Great care is required while handling the product, due to the extreme polymerization reactivity when it is exposed to insignificant amounts of ionic solutions. The delivery catheter must be rinsed internally with a non-ionic solution (e.g. water-dextrose (D5W)) before and after each use. Heparin, which is ionic, cannot be added to said solution. It is recommendable to change gloves right before using the adhesive given that they have normally been in contact with blood or physiological saline. Given that cyanoacrylates can spontaneously solidify in one hour, it is advisable to prepare the mixture right before using it.

Therefore, a possible alternative to adhesive liquid embolic agents has been developed in recent years. These alternatives are the mentioned precipitating liquid embolic agents. They consist of a polymer dissolved in a water-miscible solvent, generally DMSO. In contact with blood, the solvent spreads and the polymer coagulates due to its lack of solubility with the medium, the region thus being embolized (region refers to the specific space to be embolized). The first coagulation-inducing embolic agent to be approved was the liquid embolic agent Ethylene-Vinyl-Alcohol (EVOH) copolymer system marketed as Onyx (developed by Microtherapeutics, INC.; Irvine, CA) (US566776), which is a combination of EVOH copolymer, DMSO and tantalum powder. This product was approved for pre-operative embolization of cerebral arteriovenous malformations, and has been used in Europe for a number of years.

EVOH is a thermoplastic polymer widely used in the food packaging industry. Its chemical structure is made up of an ethylene and vinyl-alcohol copolymer (-(CH₂-CH₂)ₘ-(CH₂-CHOH)ₙ-) which is obtained from the tautomeric monomer acetaldehyde polymerized with ethylene and vinyl acetate followed by hydrolysis. The EVOH copolymer is classified according to its mole fraction of ethylene: low ethylene content provides better barrier properties and high ethylene contents lower its processing temperature. In the industry, there are formulations in which the copolymer has between 24 and 48 % mol of ethylene. Specifically, 48 % is used in Onyx.

It is a product that was not explicitly developed for this application, but rather it has been used for years as food packaging given that is one of the polymers with the lowest oxygen permeability, which helps to prolong the shelf-life of foods. Given that its barrier properties prevent the entry of oxygen into the packaging, oxidation-induced degradation reactions are delayed.

Given that it is a radiolucent material, like the cyanoacrylates, it needs to be mixed with a radiopaque substance to make it visible. On this occasion, Lipiodol cannot be used given that is a diluting agent. It is very important to maintain the viscosity designated for its purpose in coagulation-inducing liquid embolic agents. For this reason, tantalum powder is used, providing visibility without modifying viscosity. Although the tantalum particle size is of very few microns, the entire system needs to be stirred for about 20 minutes before use to achieve perfect homogenization of the tantalum. Due to the phenomenon of gravity, there is a risk of the tantalum settling, which can cause non-visualization problems, associated with phase separation.

Different problems have arisen with the use of these embolic agents, such as sticking to the catheter and to the artery, difficulty in controlling embolization depth, visualization artifacts (cast masking effect) due to the high radiopacity of tantalum, a 'tattoo' effect in embolizations close to the skin, and homogenization time.

New generations of liquid embolic agents have emerged over time for the purpose of making up for the main deficiencies observed in Onyx. The first aspect that was approached was to incorporate the visualization agent in the structure itself. Having to stir it before use to assure homogenization was thereby eliminated.

Today, Onyx has a strong competitor. It is a liquid embolic agent in which the visualization agent is incorporated in its structure. The product goes by the name PHIL (Precipitating Hydrophobic Injectable Liquid, MicroVention; Terumo; application US2017/0216484).

PHIL would be the next benchmark of the field of liquid embolic agents. It is a non-adhesive liquid embolic agent dissolved in DMSO. The iodinated component is covalently bound to the polymer. The intention of use thereof is for the embolization of injuries in both the peripheral and the neurovascular area, including arteriovenous malformations and hypervascularized tumors.

It is a polymer designed expressly for this application. It is formed by a poly(lactic-co-glycolic acid) and poly(hydroxyethyl methacrylate) copolymer with triiodophenol incorporated in a portion of the monomers through a covalent bond.

In practice, it has been observed that PHIL was much more fragile during vessel dissection and less flexible during surgery compared to Onyx. This fragility is due to the anchoring of the triiodophenol group on a rigid base structure *per se.*

Another aspect to be assessed in long-term studies is the biodegradable nature of the polymer. The use of polymers based on polylactic and polyglycolic acid when seeking biodegradation properties is well known. In this sense, the contribution by weight of the visualization agent and its binding to the monomer units to be degraded must be taken into account. As a result, the loss in mass that the embolic agent will experience as biodegradation occurs must be assessed.

On occasions, specialists have mentioned that product visualization is not sufficient. The reason is attributed to the amount of units of visualization agent per unit of polymer. Normally, radiopaque substances are inorganic in nature. Among those most used are tantalum - which is present in some of the products used up until now as embolic agents and mentioned above -, bismuth oxide, bismuth salicylate, barium sulfate and tungsten. One drawback of these substances is the need to mix the product with the radiopaque substance before use to achieve perfect homogenization, making the process less agile and requiring more planning of the amounts to be used.

There are documents in the state of the art which disclose compounds similar to the monomers of the invention and polymers thereof, but within the field of embolic materials, however, they seek to solve different problems. Some examples are mentioned below.

WO2008054205 discloses compounds which constitute radiopaque polymer particles based on an iodine or bromine substituted radiopaque monomer having specific properties as to hydrophilicity, opacity and particle size. Said embolic material comprises spherical, homogeneous and substantially nonporous radiopaque polymer particles based on at least one hydrophilic monomer and at least one radiopaque monomer.

The object of WO2011003902 is to achieve embolic materials that can be visualized by means of various imaging techniques.

WO2012019145A1 discloses radiopaque shape memory polymers (SMP) useful for medical devices, such as embolic devices, and such that said radiopaque polymer is non-metallic.

WO2006106513A2 discloses nanoparticles not specifically provided as embolic agents, but rather as contrast agents, having a mean particle size less than 2000 nm, and preferably in the range between 15 nm and 1000 nm. Said nanoparticles comprise a polymer having pendant cleavable iodine substituted groups. The term "pendant" refers to the fact that the polymer contains iodine substituted groups which are considered side groups with respect to the main chain, such that the separation thereof from the polymer does not result in fragmentation of the polymer backbone chain.

Therefore, there is a need to provide a liquid embolic agent which provides control during injection (from both the penetrability and the reflux perspectives), with suitable and non-excessive radiopacity or visibility and safety when removing the catheter (assessing the cohesion and preventing sticking to the catheter). There is also a need to provide a liquid embolic agent with radiopacity *per se*, which is biostable and has suitable elasticity so as to prevent fragmentations.

The purpose of the present invention is to provide a liquid embolic agent which shows better performance with respect to embolization of the region to be devascularized. In particular, the purpose is to provide good control during injectability (measured as penetrability and reflux), good visualization during the procedure and safety when removing the catheter (both due to the sticking thereof and due to fragmentation of the product).

A visualization agent anchored to the final polymer structure for the purpose of achieving a radiopaque polymer *per se* is used in the present invention. A physical mixture with a radiopaque substance to make the polymer visible is possible, like in a case in the state of the art, but it has drawbacks solved by the present invention.

### DESCRIPTION OF THE INVENTION

### Definitions

In this specification the term "region" refers to the specific space to be embolized in the body.

The term "polymer" refers to any polymer system comprising from 1 to 3 different monomers, thus including homopolymers (a single monomer), copolymers (two different monomers in their structure) and/or terpolymers (three different monomers in their structure). "Radiopaque monomer" is the monomer unit comprising the fragment capable of being visualized.

"First monomer" always refers to a radiopaque monomer in the invention.

"Second monomer" refers to a radiopaque or non-radiopaque monomer in the invention. In one polymer, there can be a "second monomer" or two "second monomers".

It has been found that a formulation of a radiopaque polymer *per se* with a water-miscible organic solvent gives rise to a liquid embolic agent which is capable of solving the problems of the state of the art. This radiopaque polymer *per se* formulated at a suitable concentration, or what is the same, at a certain viscosity, is capable of effectively embolizing the region to be devascularized.

The present invention has as an object a polymer of formula (I): 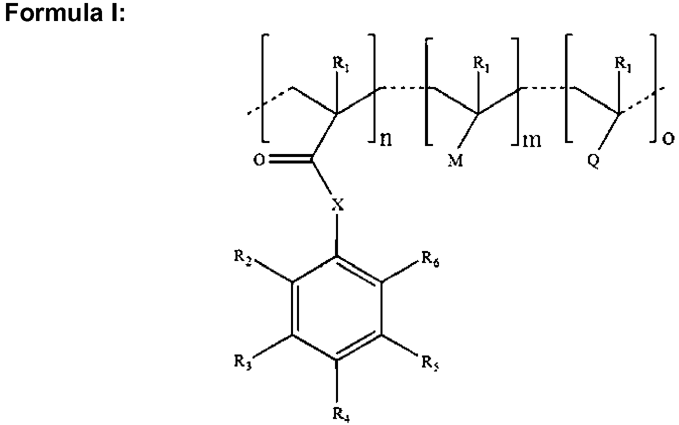 wherein:
n, m, and o ≠ 0 corresponds to a terpolymer
n≠ 0, m ≠ 0 and o =0 corresponds to a copolymer
n ≠ 0 and m = o = 0 corresponds to a homopolymer
R₁ = H, CH₃
R₂, R₃, R₄, R₅, R₆ can independently be I, Br, H
**X**= -O-; CH₂-O-; 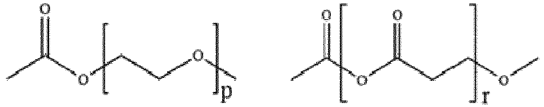 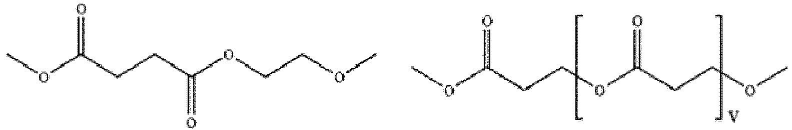 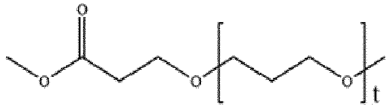
where p = 1, 6, r = 3, v = 3, and t = 6.
**M**= 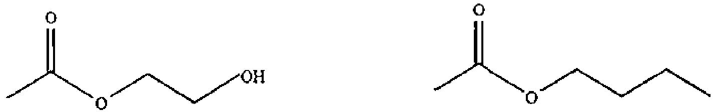 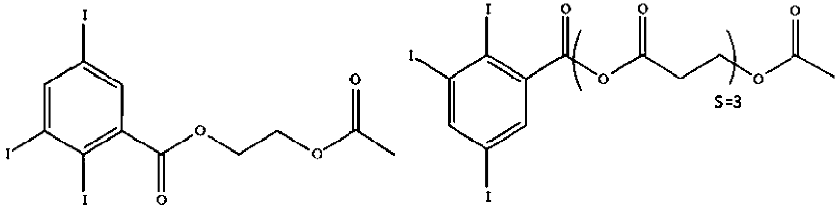 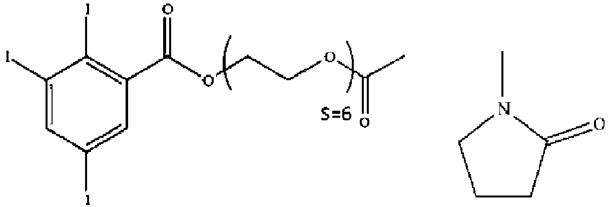
**Q=** 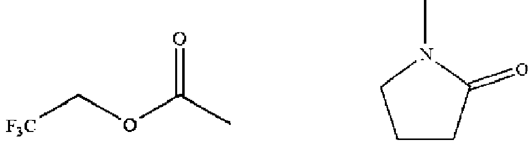
and wherein "n", "m" and "o" take values such that the molecular weight of the polymer is comprised between 50 kDa to 900 kDa.

Said polymer, as can be inferred from the values of "m", "n" and "o", can be a terpolymer, a copolymer or a homopolymer.

The molecular weight of the polymer is comprised between 50 kDa to 900 kDa. Preferably, the molecular weight ranges between 200 kDa to 800 kDa, and more preferably from 400 kDa to 600 kDa.

Particular embodiments of the polymers of the invention relate to the following polymers:

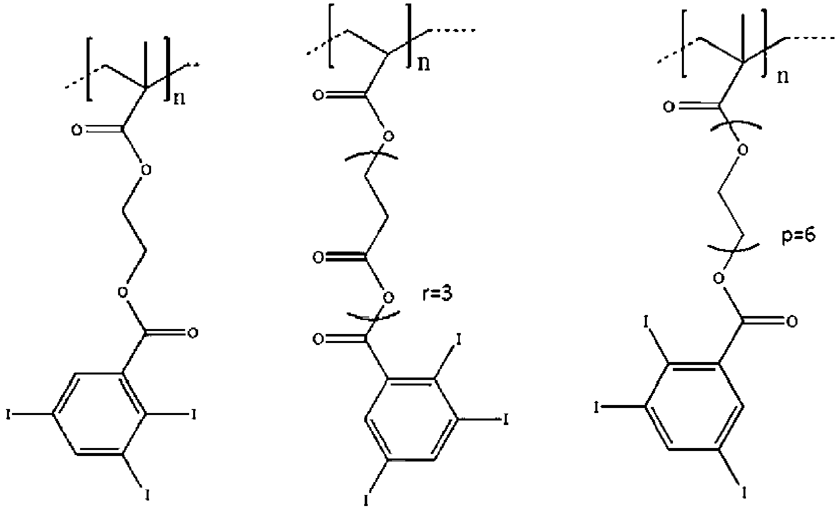

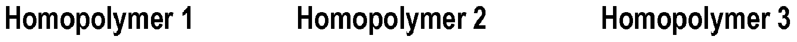

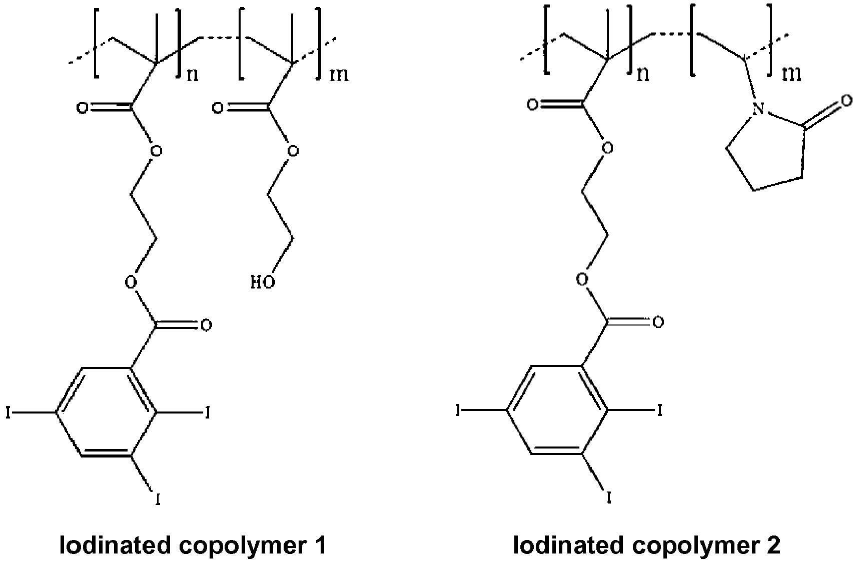

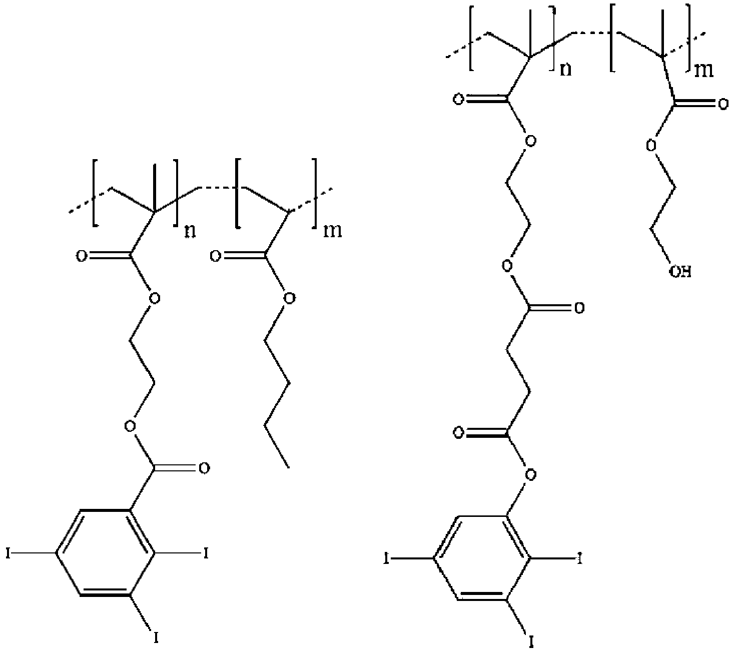

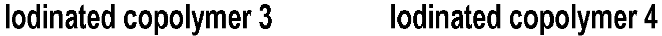

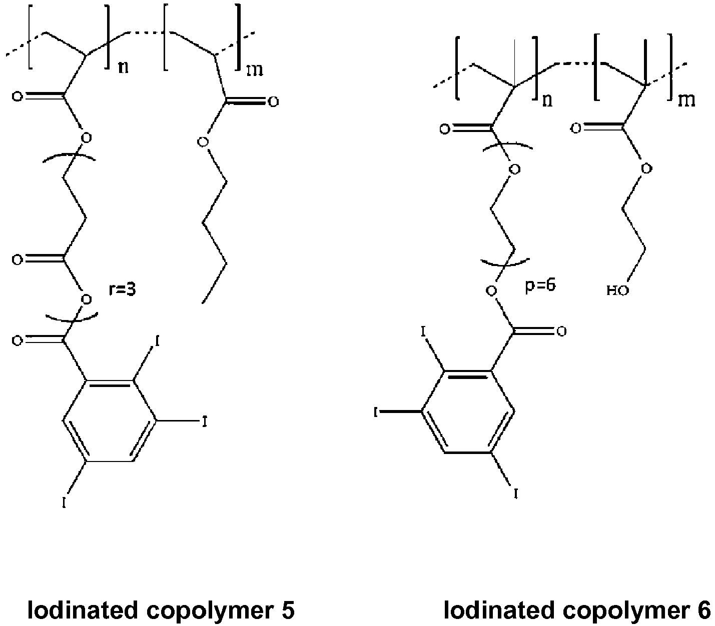

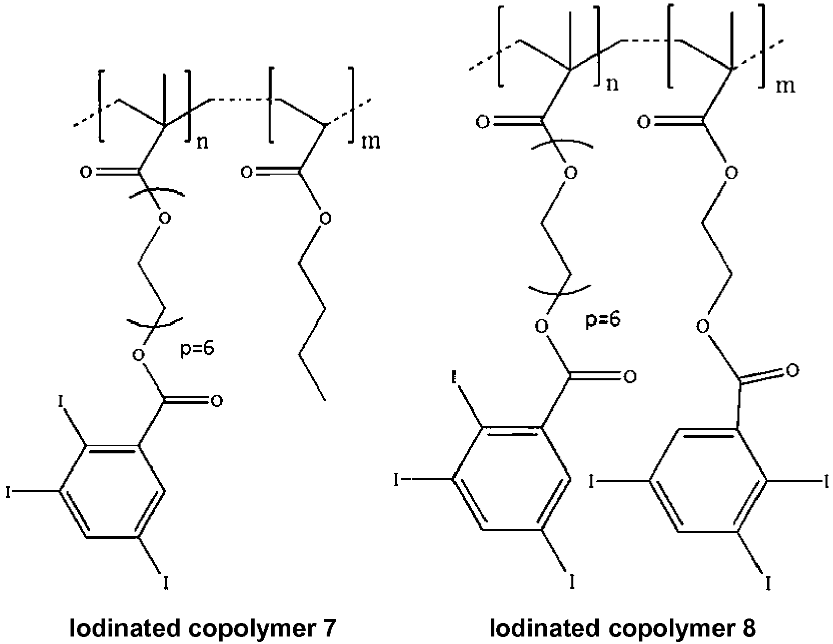

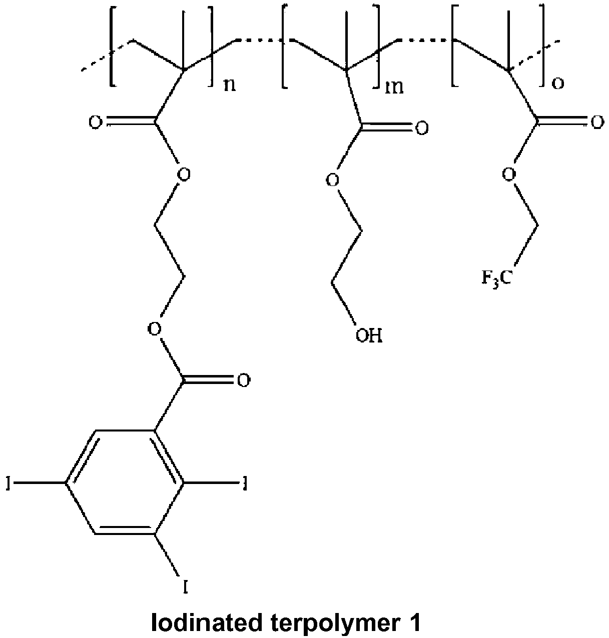

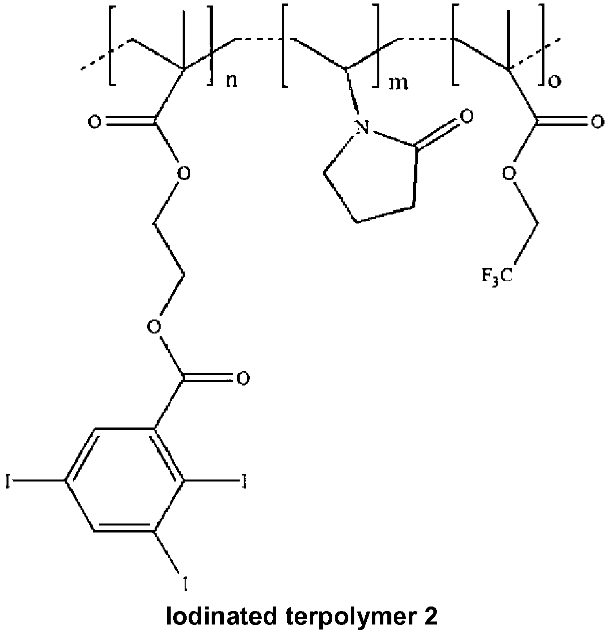

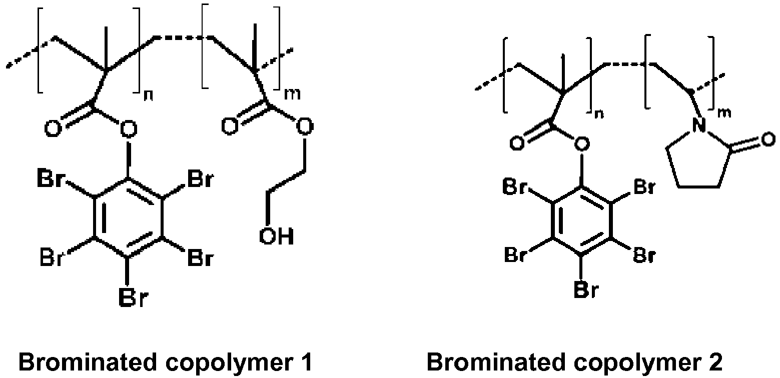

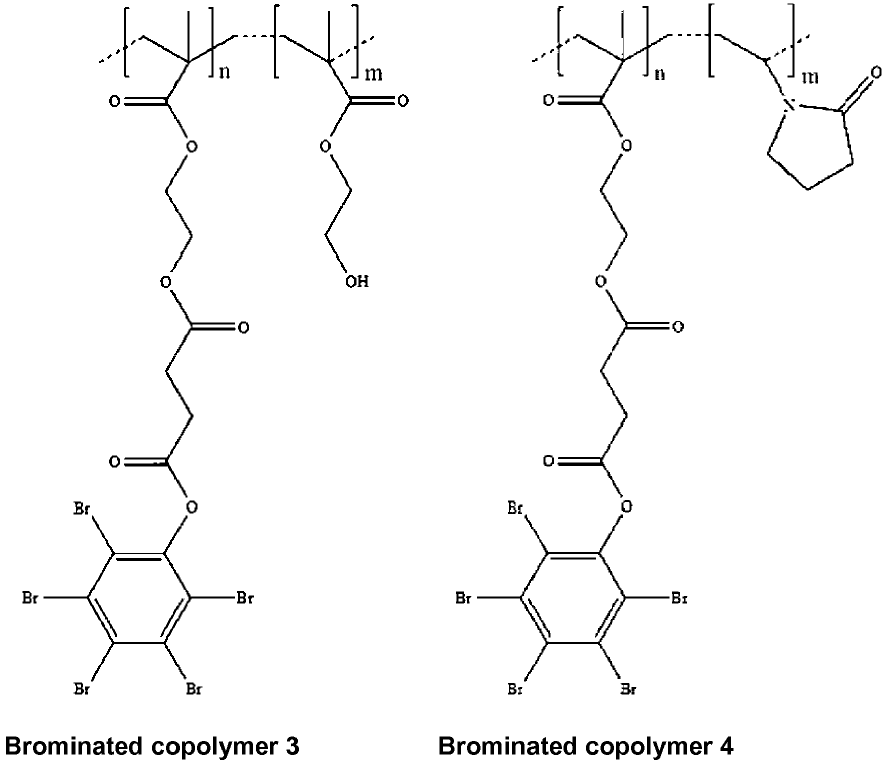

The polymer of the invention comprises at least one radiopaque monomer (referred to as "first monomer"), and it therefore contains a visualization agent which is the monomer of formula II.

The polymer of the invention can be: a homopolymer, in the event that the radiopaque monomer of formula (II) is combined with itself; a copolymer, if the radiopaque monomer is combined with a second monomer; or a terpolymer if the radiopaque monomer is combined with two different second monomers. Said second monomer can be a radiopaque or non-radiopaque monomer.

According to particular embodiments, in the case of terpolymers, said second monomers can be:
- a radiopaque monomer (of formula (II)) and a non-radiopaque monomer, or
- two different non-radiopaque monomers, or
- two different radiopaque monomers.

In the polymer of the invention, the ratio of the first monomer to the second monomer can be comprised between 40/60 and 100/0. According to preferred embodiments, the ratio is comprised between 50/50 and 100/0, and even more preferably, the ratio of the first monomer to the second monomer is 60/40.

By way of example and according to a preferred embodiment, in the poly(P3I-co-M3I) polymer the ratio of the first monomer - P3I- to the second monomer -M3I- is 60/40.

The present invention also relates to a precursor of the previously defined polymers, which is a monomer unit of formula II: 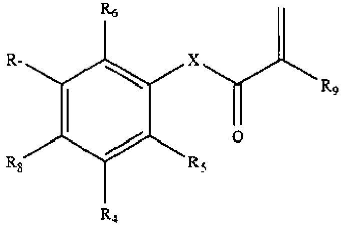 wherein:
R₄, R₅, R₆, R₇, R₈, can independently be I, Br, H
R₉ can be H, CH3
**X=** -O-; CH₂-O-; 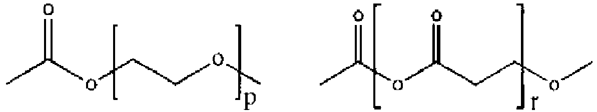 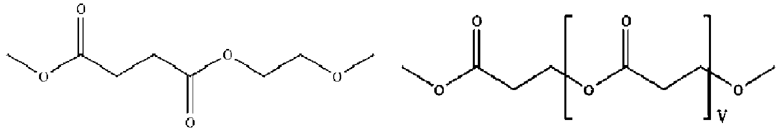 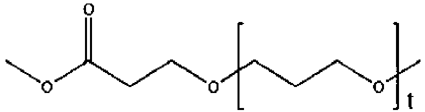
where p = 1, 6, r = 3, v = 3, and t = 6.

According to particular embodiments, the monomer of formula (II) is selected from:
**Iodinated monomer 1:** 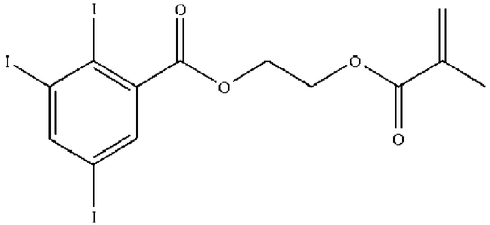
**Iodinated monomer 2:** 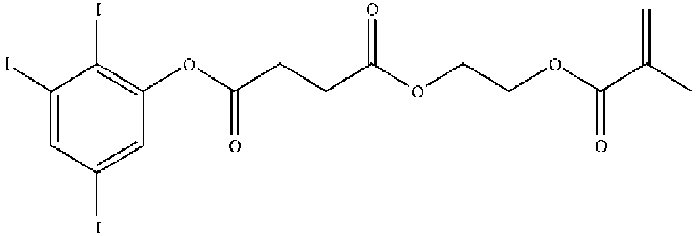
**Iodinated monomer 3:** 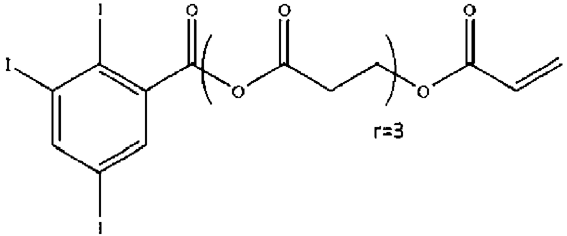
**Iodinated monomer 4:** 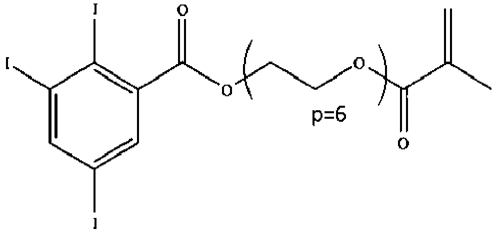
**Brominated monomer 1:** 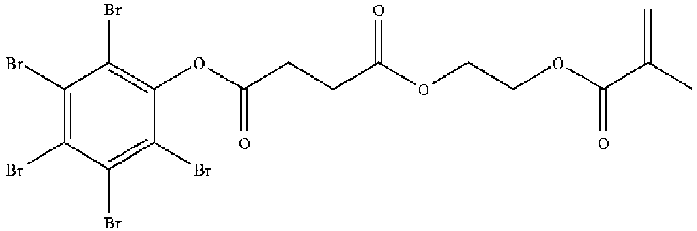
**Brominated monomer 2:** 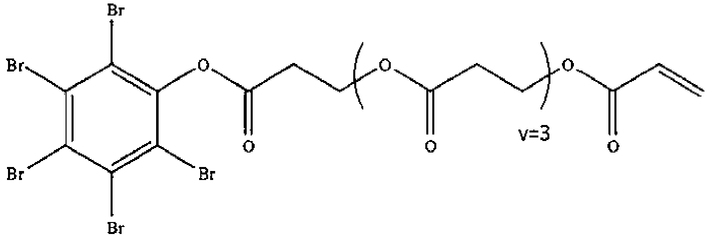
**Brominated monomer 3:**

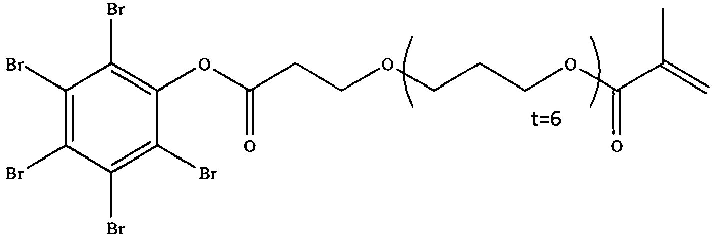

The present invention furthermore relates to a method for the preparation of the previously defined polymers. Said method comprises reacting a compound of formula II with one or two compounds selected from:
- a monomer of formula (II) (the same or different),
- 2-hydroxyethyl methacrylate (HEMA)
- n-butyl acrylate (NBA)
- 1-vinyl-2-pyrrolidinone, (VP)
- 2,2,2-trifluoroethyl methacrylate (TFEMA).

The polymerization reaction can be carried out in a water-miscible organic solvent, preferably a physiologically acceptable organic solvent, such as DMSO; for 24 hours at a temperature comprised between 55 and 65 °C, preferably at 60 °C; at a molar concentration comprised between 0.2 and 0.5 M, more preferably between 0.3 and 0.35 M; with a concentration of thermal initiator AIBN comprised between 0.01 and 0.03 M, preferably 0.015 M.

The concentration of the biocompatible polymer in the water-miscible organic solvent can be about 10 % to 60 %, from 20 % to 50 %, from 30 % to 40 %.

The liquid embolic agent can be sterilized by an autoclave or dry heat process without affecting the properties of the polymer.

The present invention has as an additional object a liquid embolic agent *per se* comprising a radiopaque polymer (terpolymer/copolymer/homopolymer) *per se*, as previously defined, solubilized in a water-miscible organic solvent, preferably a physiologically acceptable organic solvent, and more preferably DMSO.

The formulation of the liquid embolic agent can be found in a vial that can be extracted with the aid of a needle and syringe. Additionally, the syringe can subsequently be connected to a microcatheter for administration. To prevent the liquid embolic agent from solidifying inside the catheter, the dead volume of the catheter is usually filled with the same amount by volume of the water-miscible organic solvent. This procedure prevents the obstruction of the delivery/administration catheter with the embolic polymer. Alternatively, the formulation of the polymer of the liquid embolic agent can be pre-filled in a delivery syringe. The syringe can be connected directly to the proximal end of the delivery/administration catheter, such as a microcatheter, cannula or the like, and be delivered to the vascular site or other desired anatomical site.

The invention also relates to a method for producing a liquid embolic agent comprising a radiopaque polymer (terpolymer/copolymer/homopolymer) *per se*, which method comprises the steps of synthesizing the monomer unit which will contain the visualization agent by means of a covalent bond; polymerizing until obtaining the radiopaque polymer *per se* and finally formulating with a polymer-solubilizing and water-miscible organic solvent.

### Use of the liquid embolic agent of the invention

The microcatheters used for procedures of this type must be compatible with the solvent used, for example, DMSO. Otherwise, DMSO may cause damage to some parts of the microcatheter, preventing its correct working and causing problems during the intervention.

Unlike what has been observed with Onyx, there is no risk of entrapment of the microcatheter by the liquid embolic agent after use. This prevents risks of damage to the arterial wall and the need to work with a microcatheter with a detachable tip.

In the procedure, the delivery/administration device (microcatheter) is inserted in the vessel to be treated; the delivery/administration device is guided towards the area in need of treatment where physiological medium can be found; the liquid embolic agent is injected through the delivery/administration device into the area in need of treatment, the polymer thus immediately precipitating and forming a solid polymeric mass, with the vascular region being thus treated. A liquid-to-solid transition occurs at the delivery site as it is subjected to physiological conditions.

The liquid embolic agent of the invention includes viewable agents chemically anchored to its structure, which impart visualization of the polymer when it is observed using any imaging technique such as fluoroscopy, computed tomography or magnetic resonance techniques.

The visualization agents are included in the polymer once polymerization has concluded. This visualization is imparted by monomers containing halogenated groups in their central structure, particularly an aromatic ring with large amount of halogens, particularly bromine and iodine, and more particularly iodine. The concentration of iodine for making it viewable under any imaging technique ranges from 10 % to around 60 % w/w (by mass, total 100 g polymer/g); from 20 % to 50 %, from 30 % to 40 % w/w.

### Advantages of the liquid embolic agents of the invention

The liquid embolic agent of the invention is surprisingly effective, allowing control of injectability, penetration and reflux, such that there is control over the advance of the liquid during the injection thereof and the stopping thereof when it is not injected. At the same time, this formulation allows visualization and tracking of the liquid during the entire embolization procedure in standard working equipment. In turn, the removal of the catheter is safe, eliminating the risks associated with sticking to the catheter. The product is cohesive, whereby eliminating the risk of fragmented migration.

Additionally, the liquid embolic agent shows high patient safety because the radiopaque polymer *per se* according to the invention does not stick to the catheter, is very cohesive and is harmless for humans, and therefore, non-toxic. Cohesive forces between chains of monomer units are improved, which in turn determines the flexibility of the polymer, glass transition temperature, melting temperature and crystallization capacity, among other properties. Additionally, the polymers have the surprising effect of completely filling the region providing correct embolization. It has been observed that in the different periods of study after the procedure, occlusion of the vessel by arteriography is permanent, without observing recanalization thereof. The region to be treated can be any vascular malformation.

In particular, the hydrocarbon chains in the monomers create a slightly hydrophilic environment which controls the strong hydrophobic character of the aromatic ring containing the halogenated groups responsible for the radiopaque properties. Additionally, due to this composition, the polymer is also slightly hydrophilic, such that the coagulation speed/time in contact with the blood is modulated, allowing control of penetration and reaching more distal regions compared to other products of the state of the art.

The monomer unit ratio by weight is important for controlling the visualization and cohesion properties of the radiopaque polymer *per se.* If the first monomer/second monomer ratio by weight of the radiopaque polymer *per se* is comprised between previously indicated ranges, it obtains a good balance of penetration, visualization and cohesion properties, which are the three requirements with which the polymer must comply. However, an excellent balance between control of penetration and maximum visualization during the embolization procedure is achieved with a first monomer/second monomer ratio by weight of 60/40 to 100/0. In contrast, if a first monomer/second monomer ratio by weight of less than 40/60 is used, it increases the risk of a lack of control of penetration since a decrease in product cohesion is observed; and the loss of visualization during embolization is observed since the amount of radiopaque agent is insufficient for achieving correct tracking of the product.

### Molecular weight

The molecular weight of the radiopaque polymer used in the radiopaque liquid embolic agent *per se* is included in the indicated ranges, which allows obtaining high treatment efficacy. Normally, molecular weight is expressed in kilodaltons (kDa). A polymer having a very small molecular weight penetrates too much, and if it is too large, the polymer does not penetrate enough. With this molecular weight, high treatment efficacy is obtained and the possible lack of control and possible lack of cohesion are significantly reduced.

### Viscosity

Besides the molecular weight, the concentration of the radiopaque polymer *per se* in the formulation and, accordingly, the viscosity, is also important. The radiopaque liquid embolic agent can exist in different concentrations. Normally, the concentration is expressed as the percentage of non-volatile components (%NV) (%NV = (mNV/mT)*100, where mNV = weight of all the non-volatile components; mT = weight of all the components (volatile and non-volatile). In general, there can be three types of scenarios to be embolized depending on the flow that may exist: low, moderate and high associated with the type of vascular area to be treated. In the present invention, %NV can comprise values of 10 % to 60 %. Preferably, %NV ranges between 20 % to 50 %, and more preferably from 25 % to 35 %. With the viscosities (which correspond to these percentages of non-volatile components) which are achieved in the present invention, the possibility of embolizing any vascular region depending on the flow is covered.

That which has been described comprises several embodiments by way of example. It is understood that one having ordinary skill in the art would derive these different permutations and possible combinations of the different embodiments and aspects that are described after a direct and objective reading of this disclosure. One having ordinary skill in the art would understand that the description of the embodiments that is presented does not limit the invention, nor do the drawings.

### Brief Description of the Figures

FIG. 1 are images corresponding to the coagulation of the liquid embolic agent in Simulated Body Fluid (SBF), according to the present invention.
FIG. 2 shows the claimed liquid embolic agent precipitated in SBF.
FIG. 3 shows angiographic images of the rete mirabile of a porcine animal model before (left) and after (right) embolization with a liquid embolic agent according to the invention. The stained areas correspond to non-embolized regions, showing the passage of the contrast liquid.
FIG. 4 shows angiographic images of a radiopaque liquid embolic agent *per se* during embolization of the rete mirabile of a porcine model (left) and after embolization (right). The stained areas correspond to the presence of the liquid embolic agent according to the present invention.

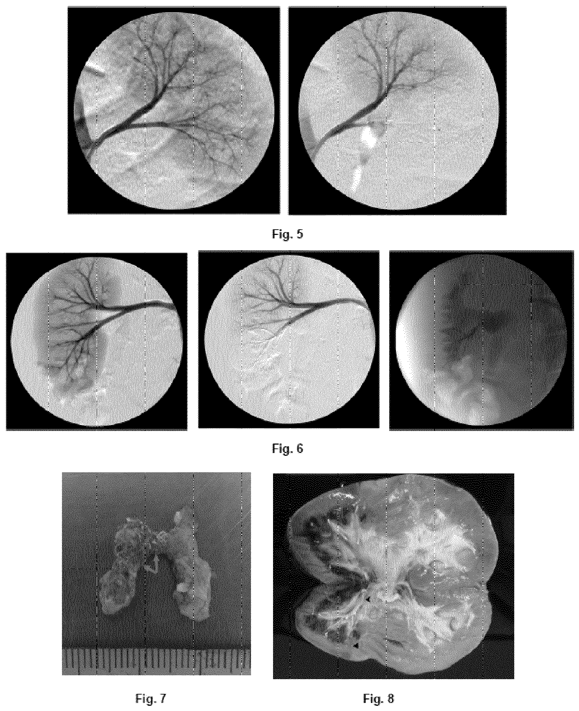

FIG. 5 shows angiographic images of the left kidney of a porcine animal model before embolization (left) and after embolization of the lower pole (right). The black stained areas correspond to non-embolized regions, showing the passage of the contrast liquid.
FIG. 6 shows angiographic images of the right kidney of a porcine model before embolization (left), after embolization with a liquid embolic agent according to the invention (center) and the radiopacity thereof 30 days after the embolization procedure (right). The black stained areas in the left and central images correspond to non-embolized regions, where the contrast liquid can circulate.
FIG. 7 shows a macroscopic image of the rete mirabile of a porcine model 24 hours after embolization with a liquid radiopaque per se, according to the invention. The right side was used as a control with contrast liquid.
FIG. 8 depicts the section of the right kidney of a porcine model 24 hours after embolization with an embolic agent according to the invention. The presence of the claimed liquid embolic agent in the polar artery (<), interlobular arteries (†) and arcuate arteries (◄) can be seen.

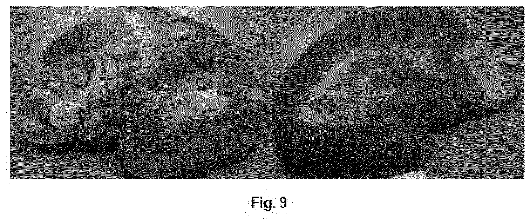

FIG. 9 corresponds to the sagittal section of the left kidney of a porcine model 30 days after embolization of the lower pole with a liquid embolic agent with intrinsic radiopacity. In the procedure, the lower pole of the kidney was selectively embolized with the claimed liquid embolic agent.

### EXAMPLES

### Synthesis of radiopaque monomers

### Iodinated monomer 1: M3I

2,3,5-triiodobenzoic acid (TIBA) (1.0 eq.) and 2-hydroxyethyl methacrylate (HEMA) (1.2 eq.) in 200 ml of diethyl ether were added to a round-bottom flask. Dicyclohexylcarbodiimide (DCC) (1.1 eq.) and dimethylaminopyridine (DMAP) (0.1 eq.) were dissolved in the same solvent and incorporated drop-wise into the reaction under constant stirring at room temperature. Previously, the HEMA was purified according to the standard procedure described in the literature (European Polymer Journal, 1976, 12, 685-9).

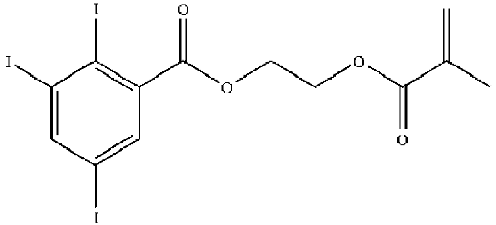

The reaction mixture was maintained under the same conditions for 24 hours. The reaction mixture was subsequently filtered to remove dicyclohexylurea (DCU) which precipitates during the reaction. The organic phase was subsequently washed 3 times with solutions of sodium hydroxide (NaOH) (0.1 N) and hydrochloric acid (HCl) (0.1 N). The organic phase was then washed with a saturated solution of sodium bicarbonate (NaHCO₃) and with water. Finally, the resulting mixture was dried with anhydrous sodium sulfate (NaₛSO₄), filtered, and the solvent was removed under reduced pressure.

### Iodinated monomer 2: M3Iext

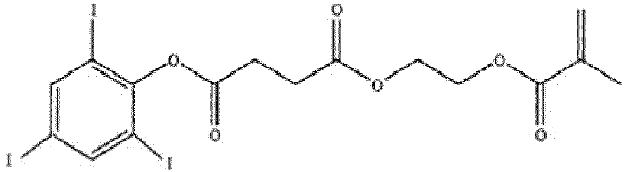

2,4,6-Triiodophenol (1 eq.) and the spacer mono-2-(methacryloyloxy)ethylsuccinate (MES) (1.2 eq.) in diethyl ether (about 300 ml for 10 g of 2,4,6-triidophenol) were dissolved in a round-bottom flask. The solution was maintained under stirring and under nitrogen atmosphere. On one hand, DCC (1.2 eq.) and DMAP (0.2 eq.) were dissolved in the same solvent and incorporated drop-wise into the reaction under constant stirring at room temperature and under nitrogen atmosphere.

The reaction mixture was maintained under constant stirring for 24 hours. After this time elapsed, the reaction mixture was filtered to remove DCU which precipitates during the reaction.

The organic phase was subsequently washed 3 times with solutions of NaOH (0.1 N) and HCl (0.1 N). The organic phase was then washed 3 times with a saturated solution of NaHCO₃ and with water. The resulting mixture was dried with anhydrous Na₂SO₄, filtered, and the solvent was removed under reduced pressure.

### Iodinated monomer 3: Acri3I

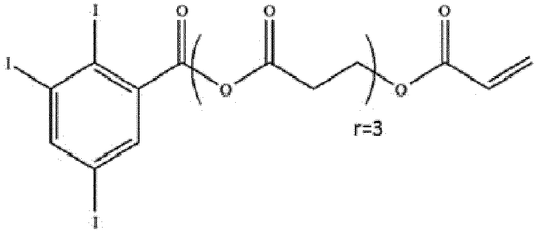

TIBA (1.1 eq.) and the acrylate spacer (2-carboxyethyl acrylate oligomers, n=3, 1 eq) in 250 ml of a mixture of chloroform and diethyl ether (50:50 v:v) were added to a round-bottom flask. DCC (1.2 eq.) and DMAP (0.1 eq.) were dissolved in the same solvent and incorporated drop-wise into the reaction under constant stirring at room temperature.

The reaction mixture was maintained under the same conditions for 24 hours. The reaction mixture was subsequently filtered to remove DCU which precipitates during the reaction. The organic phase was subsequently washed 3 times with solutions of NaOH (0.1 N) and HCl (0.1 N). The organic phase was then washed with a saturated solution of NaHCO₃ and with water. Finally, the resulting mixture was dried with anhydrous Na₂SO₄, filtered, and the solvent was removed under reduced pressure.

### Iodinated monomer 4: P3I

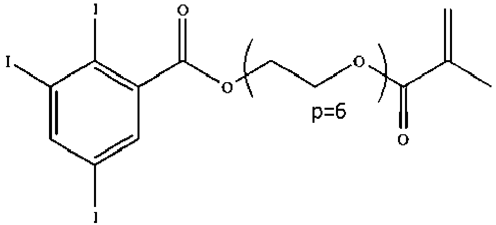

TIBA (1.2 eq.) and the spacer polyethyleneglycol methacrylate (PEGMA) (1 eq., average molecular weight 360 Da) in a mixture of diethyl ether and tetrahydrofuran (THF) (50:50 v:v, about 150 ml of each solvent for 25 g of TIBA) were dissolved in a round-bottom flask. The solution was maintained under stirring and under nitrogen atmosphere. DCC (1.1 eq.) and DMAP (0.1 eq.) were then dissolved in the same solvent and incorporated drop-wise into the reaction under constant stirring at room temperature and under nitrogen atmosphere.

The reaction mixture was maintained under constant stirring for 24 hours. After this time elapsed, the reaction mixture was filtered to remove DCU which precipitates during the reaction. The solvent was removed under reduced pressure and the product was resuspended in 50 ml of diethyl ether. This mixture was placed in the freezer for 24h to favor precipitation of DCU.

After 24 h, the reaction mixture was cold filtered and the product resuspended in diethyl ether was frozen again. After several hours, the reaction medium was filtered and washed 3 times with solutions of NaOH (0.1 N) and HCl (0.1 N). The organic phase was then washed 3 times with a saturated solution of NaHCO₃ and with water. After removing the solvent, the resulting mixture was resuspended again in 50 ml of diethyl ether and placed in the freezer for several hours.

Finally, the mixture was cold filtered, washed once with a saturated solution of NaHCO₃, dried with anhydrous Na₂SO₄, filtered, and the product was isolated removing the solvent under reduced pressure.

### Brominated monomer 1: M5Brext

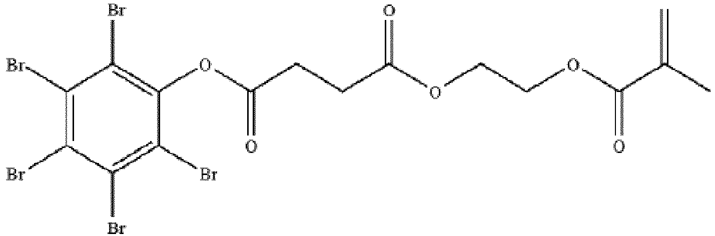

Pentabromophenol (5BrPh) (1 eq.) and the spacer MES (1.2 eq.) in diethyl ether (about 200-250 ml for 8 g of 5BrPh) were dissolved in a round-bottom flask. The solution was maintained under stirring and under nitrogen atmosphere. On one hand, DCC (1.2 eq.) and DMAP (0.2 eq.) were dissolved in the same solvent and incorporated drop-wise into the reaction under constant stirring at room temperature and under nitrogen atmosphere.

The reaction mixture was maintained under constant stirring for 24 hours. After this time elapsed, the reaction mixture was filtered to remove DCU which precipitates during the reaction.

The organic phase was subsequently washed 3 times with solutions of NaOH (0.1 N) and HCl (0.1 N). The organic phase was then washed 3 times with a saturated solution of NaHCO₃ and with water. The resulting mixture was dried with anhydrous Na₂SO₄, filtered, and the solvent was removed under reduced pressure, obtaining a white solid. Finally, the obtained product was washed with ethanol for the purpose of removing excess unreacted spacer MES, and the solid was dried under reduced pressure.

### Brominated monomer 2: Acri5Br

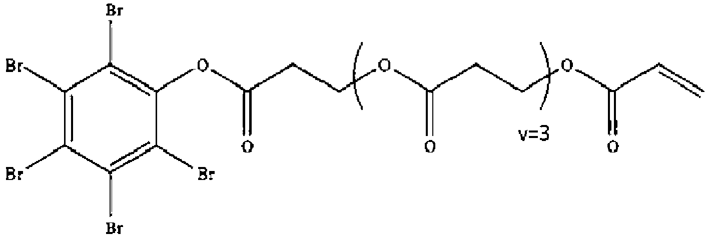

Pentabromophenol (5BrPh) (1.1 eq.) and the spacer acrylate (2-carboxyethyl acrylate oligomers, n=3, 1 eq) in 250 ml of a mixture of chloroform and diethyl ether (50:50 v:v) were dissolved In a round-bottom flask. The solution was maintained under stirring and under nitrogen atmosphere. On one hand, DCC (1.2 eq.) and DMAP (0.2 eq.) were dissolved in the same solvent and incorporated drop-wise into the reaction under constant stirring at room temperature and under nitrogen atmosphere.

The reaction mixture was maintained under the same conditions for 24 hours. The reaction mixture was subsequently filtered to remove DCU which precipitates during the reaction. The organic phase was subsequently washed 3 times with solutions of NaOH (0.1 N) and HCl (0.1 N). The organic phase was then washed with a saturated solution of NaHCO₃ and with water. Finally, the resulting mixture was dried with anhydrous Na₂SO₄, filtered, and the solvent was removed under reduced pressure.

### Brominated monomer 3: P5Br

Pentabromophenol (5BrPh) (1 eq.) and the spacer PEGMA (1.2 eq.) in diethyl ether were dissolved in a round-bottom flask. The solution was maintained under stirring and under nitrogen atmosphere.

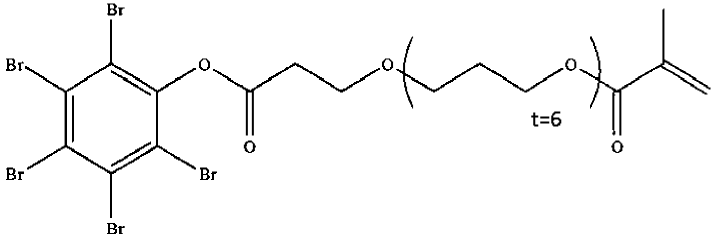

On one hand, DCC (1.2 eq.) and DMAP (0.2 eq.) were dissolved in the same solvent and incorporated drop-wise into the reaction under constant stirring at room temperature and under nitrogen atmosphere.

The reaction mixture was maintained under constant stirring for 24 hours. After this time elapsed, the reaction mixture was filtered to remove DCU which precipitates during the reaction.

The organic phase was subsequently washed 3 times with solutions of NaOH (0.1 N) and HCl (0.1 N). The organic phase was then washed 3 times with a saturated solution of NaHCO₃ and with water. The resulting mixture was dried with anhydrous Na₂SO₄, filtered, and the solvent was removed under reduced pressure.

### Synthesis of radiopaque polymers

### Iodinated homopolymer 1: poly(M3I)

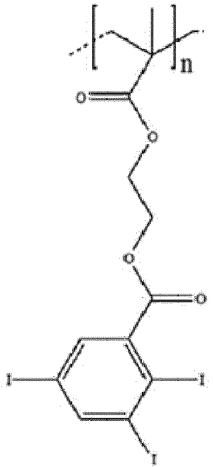

The poly(M3I) homopolymers were prepared by radical polymerization of M3I methacrylic monomer in solution and at high conversion.

For carrying out the different polymerizations, M3I monomer was weighed and dissolved in a Pyrex container having a capacity suitable for carrying out polymerization. The necessary volume of anhydrous DMSO was then added and it was necessary to wait for complete dissolution of the monomer. The necessary amount of 2-2'- azobisisobutyronitrile (AIBN) was subsequently added as a polymerization initiator.

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath. The solid was then left to decant in the refrigerator for 2-3 h. Finally, the obtained precipitate was washed using a centrifuge (10000 rpm cycles for 5 min) with water 3 times to remove DMSO. Finally, the obtained product was isolated by lyophilization.

Following this methodology, different homopolymerization reactions were prepared by varying the molarity of the solution (M) and the concentration of AIBN.

### Iodinated homopolymer 2: poly(Acri3I)

The poly(Acri3I) homopolymers were prepared by radical polymerization of Acri3I acrylic monomer in solution and at high conversion.

For carrying out the different polymerizations, Acri3I monomer was weighed and dissolved in a Pyrex container having a capacity suitable for carrying out polymerization. The necessary volume of anhydrous DMSO was then added and it was necessary to wait for complete dissolution of the monomer. The necessary amount of AIBN was subsequently added as a polymerization initiator.

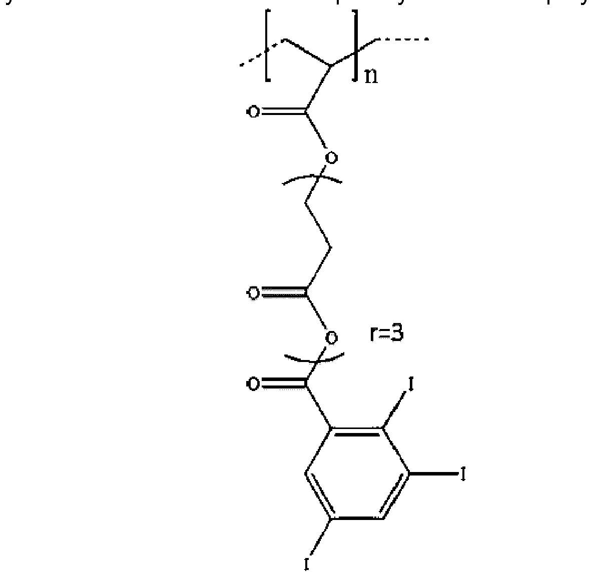

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath. Finally, the obtained precipitate was washed using a centrifuge (10000 rpm cycles for 5 min) with water 3 times to remove DMSO. The obtained product was isolated by lyophilization.

Following this methodology, different homopolymerization reactions were prepared by varying the molarity of the solution (M) and the concentration of AIBN.

### Iodinated homopolymer 3: poly(P3I)

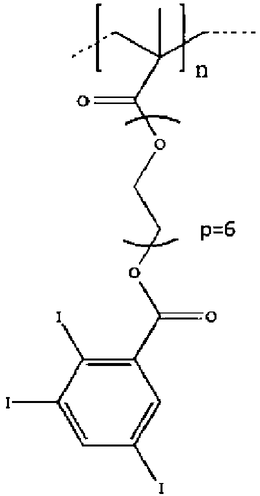

The poly(P3I) homopolymers were prepared by radical polymerization of P3I methacrylic monomer in solution and at high conversion.

For carrying out the different polymerizations, P3I monomer was weighed and dissolved in a Pyrex container having a capacity suitable for carrying out polymerization. The necessary volume of anhydrous DMSO was then added and it was necessary to wait for complete dissolution of the monomer. The necessary amount of AIBN was subsequently added as a polymerization initiator.

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath, obtaining a gummy precipitate which can be readily isolated from the water used for precipitation. Finally, the obtained precipitate was washed using a centrifuge (10000 rpm cycles for 5 min) with water 3 times to remove DMSO. Finally, the obtained product was isolated by lyophilization.

Following this methodology, different homopolymerization reactions were prepared by varying the molarity of the solution (M) and the concentration of AIBN.

### Iodinated copolymer 1: poly(M3I-co-HEMA)

The poly(M3I-co-HEMA) copolymers were prepared by radical polymerization of the methacrylic monomer derived from TIBA (M3I) with HEMA in solution and at high conversion. Previously, the HEMA was purified according to the standard procedure described in the literature *(*European Polymer Journal, 1976, 12, 685-9).

To that end, the M3I and HEMA monomers were weighed and dissolved in a Pyrex container having a capacity suitable for carrying out polymerization. The volume of anhydrous DMSO was then added and it was necessary to wait for complete dissolution of the comonomers of the reaction. The necessary amount of AlBN was subsequently added as a polymerization initiator.

The reaction mixture was deoxygenated under nitrogen (N₂) atmosphere for 5 minutes and maintained in an oven for 24 hours at 60 °C, without stirring. Once the polymerization time elapsed, the reaction mixture was cooled to stop polymerization. The solution was subsequently precipitated on water with vigorous stirring in an ice bath. The obtained precipitate was then washed with water 3 times to remove DMSO and another 3 times with ethanol while hot to remove the unreacted iodinated methacrylic derivative. Finally, the obtained product was isolated by lyophilization.

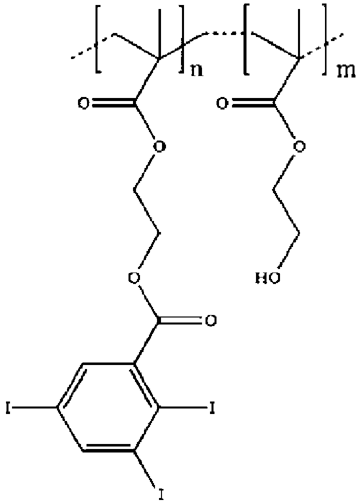

Following this methodology, different polymerization reactions were prepared by varying the molar composition in the comonomer feed, the molarity of the solution (M) and the concentration of AIBN.

### Iodinated copolymer 2: poly(M3I-co-VP)

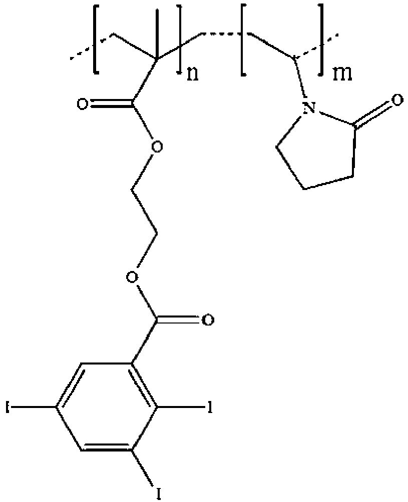

The poly(M3I-co-VP) copolymers were prepared by radical polymerization of M3I methacrylic monomer with VP in solution and at high conversion. Previously, the VP was purified by vacuum distillation. To that end, the M3I and VP monomers were weighed and dissolved in a Pyrex container having a capacity suitable for carrying out polymerization.

The necessary volume of anhydrous DMSO was then added and it was necessary to wait for complete dissolution of the comonomers of the reaction. The necessary amount of AIBN was subsequently added as a polymerization initiator.

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath. The solid was then left to decant in the refrigerator for 2-3 h. Finally, the obtained precipitate was washed using a centrifuge (10000 rpm cycles for 5 min) with water 3 times to remove DMSO and another 3 times with ethanol while hot to remove the unreacted iodinated methacrylic derivative. Finally, the obtained product was isolated by lyophilization.

Following this methodology, different polymerization reactions were prepared by varying the molar composition in the comonomer feed, the molarity of the solution (M) and the concentration of AIBN.

### Iodinated copolymer 3: poly(M3I-co-NBA)

The poly(M3I-co-NBA) copolymers were prepared by radical polymerization of the M3I methacrylic monomer with NBA in solution and at high conversion. To that end, the M3I and NBA monomers were weighed and dissolved in a Pyrex container having a capacity suitable for carrying out polymerization.

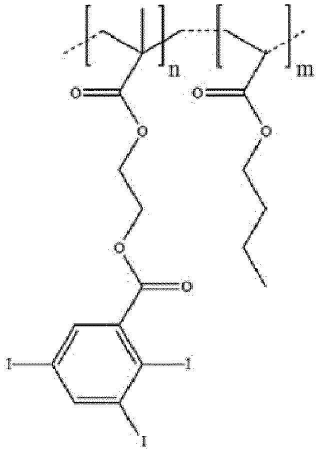

The necessary volume of anhydrous DMSO was then added and it was necessary to wait for complete dissolution of the comonomers of the reaction. The necessary amount of AIBN was subsequently added as a polymerization initiator. The reaction mixture was deoxygenated under N₂ atmosphere for 5 minutes and maintained in an oven for 24 hours at 60 °C, without stirring.

Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath. The solid was then left to decant in the refrigerator for 2-3 h. Finally, the obtained precipitate was washed using a centrifuge (10000 rpm cycles for 5 min) with water 3 times to remove DMSO. Finally, the obtained product was isolated by lyophilization.

Following this methodology, different polymerization reactions were prepared by varying the molar composition in the comonomer feed, the molarity of the solution (M) and the concentration of AIBN.

### Iodinated copolymer 4: poly(M3Iext-co-HEMA)

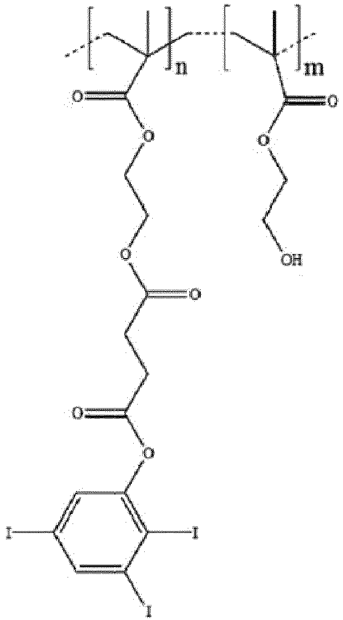

The poly(M3Iext-co-HEMA) copolymers were prepared by radical polymerization of the M3Iext methacrylic monomer with HEMA in solution and at high conversion. Previously, the HEMA was purified according to the standard procedure described in the literature (European Polymer Journal, 1976, 12, 685-9).

To that end, the monomers were weighed and dissolved in a Pyrex container having a capacity suitable for carrying out polymerization. The necessary volume of anhydrous DMSO was then added and it was necessary to wait for complete dissolution of the comonomers of the reaction. The necessary amount of AIBN was subsequently added as a polymerization initiator.

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath. The solid was then left to decant in the refrigerator for 2-3 h. Finally, the obtained precipitate was washed using a centrifuge (10000 rpm cycles for 5 min) with water 3 times to remove DMSO and another 3 times with ethanol while hot to remove the unreacted iodinated methacrylic derivative. Finally, the obtained product was isolated by lyophilization.

Following this methodology, different polymerization reactions were prepared by varying the molar composition in the comonomer feed, the molarity of the solution (M) and the concentration of AIBN.

### Iodinated copolymer 5: poly(Acri3I-co-NBA)

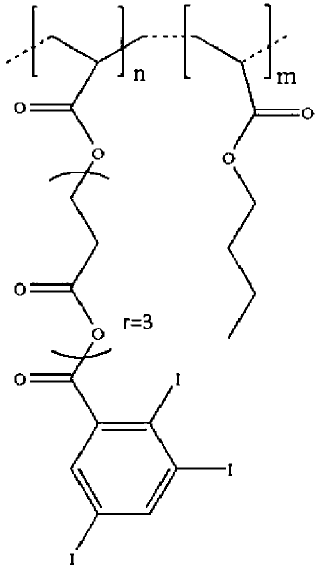

The poly(Acri3I-*co*-NBA) copolymers were prepared by radical polymerization of the Acri3I acrylic monomer with NBA in solution and at high conversion.

To that end, the Acri3I and NBA monomers were weighed and dissolved in a Pyrex container having a capacity suitable for carrying out polymerization. The necessary volume of anhydrous DMSO was then added and it was necessary to wait for complete dissolution of the comonomers of the reaction. The necessary amount of AlBN was subsequently added as a polymerization initiator.

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath. The solid was then left to decant in the refrigerator for 2-3 h. Finally, the obtained precipitate was washed using a centrifuge (10000 rpm cycles for 5 min) with water 3 times to remove DMSO. Finally, the obtained product was isolated by lyophilization.

Following this methodology, different polymerization reactions were prepared by varying the molar composition in the comonomer feed, the molarity of the solution (M) and the concentration of AlBN.

### Iodinated copolymer 6: poly(P31-co-HEMA)

The poly(P3I-co-HEMA) copolymers were prepared by radical polymerization of the P3I methacrylic monomer and HEMA in solution and at high conversion. Previously, the HEMA was purified according to the standard procedure described in the literature (European Polymer Journal, 1976, 12, 685-9).

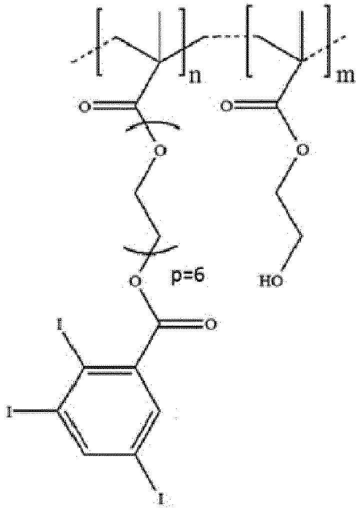

For carrying out the different polymerizations, the P3I and HEMA comonomers were weighed and dissolved in a Pyrex container having a capacity suitable for carrying out polymerization. The necessary volume of anhydrous DMSO was then added and it was necessary to wait for complete dissolution of the comonomers of the reaction. The necessary amount of AlBN was subsequently added as a polymerization initiator.

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath, obtaining a gummy precipitate which can be readily isolated from the water used for precipitation. Finally, the obtained precipitate was washed using a centrifuge (10000 rpm cycles for 5 min) with water 3 times to remove DMSO. Finally, the obtained product was isolated by lyophilization.

Following this methodology, different polymerization reactions were prepared by varying the molar composition in the comonomer feed, the molarity of the solution (M) and the concentration of AlBN.

### Iodinated copolymer 7: poly(P3I-co-NBA)

The poly(P3I-co-NBA) copolymers were prepared by radical polymerization of the P3I methacrylic monomer and NBA in solution and at high conversion.

For carrying out the different polymerizations, the P3I and NBA comonomers were weighed and dissolved in a Pyrex container having a capacity suitable for carrying out polymerization. The necessary volume of anhydrous DMSO was then added and it was necessary to wait for complete dissolution of the comonomers of the reaction. The necessary amount of AIBN was subsequently added as a polymerization initiator.

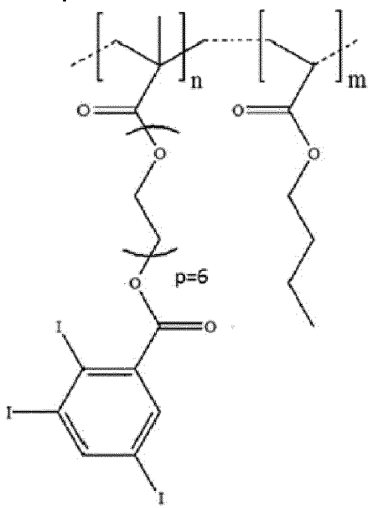

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath, obtaining a gummy precipitate which can be readily isolated from the water used for precipitation. Finally, the obtained precipitate was washed using a centrifuge (10000 rpm cycles for 5 min) with water 3 times to remove DMSO. Finally, the obtained product was isolated by lyophilization.

Following this methodology, different polymerization reactions were prepared by varying the molar composition in the comonomer feed, the molarity of the solution (M) and the concentration of AIBN.

### Iodinated copolymer 8: poly(P3I-co-M3I)

The poly(P3I-co-M3I) copolymers were prepared by radical polymerization of the synthetic methacrylic monomers derived from TIBA with HEMA (M3I) and PEGMA (P3I) (Mn = 360) in solution and at high conversion.

For carrying out the different polymerizations, the comonomers were weighed and dissolved in a Pyrex container having a capacity suitable for carrying out polymerization. The necessary volume of anhydrous DMSO was then added and it was necessary to wait for complete dissolution of the comonomers of the reaction. The necessary amount of AIBN was subsequently added as a polymerization initiator.

The reaction mixture was deoxygenated under N₂ atmosphere for 5 minutes and was maintained in an oven for 24 h a 60 °C, without stirring. Once the reaction time elapsed, the reaction mixture was cooled to stop polymerization.

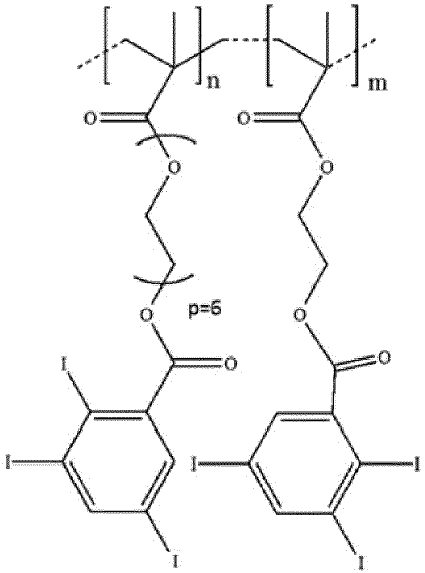

The solution was subsequently precipitated on water (in a 2 l-capacity beaker with about 1.0-1.5 l of water) with vigorous stirring in an ice bath. The solid was then left to decant in the refrigerator for 2-3 h. Next, the obtained precipitate was washed using a centrifuge (8000 rpm cycles for 5 min) with water 3 times to remove DMSO. Finally, the obtained product was isolated by lyophilization.

Following this methodology, different polymerization reactions were prepared by varying the molar composition in the comonomer feed, the molarity of the solution (M) and the concentration of AIBN.

### Iodinated terpolymer 1: poly(M3I-co-HEMA-co-TFEMA)

The poly(M3I-co-HEMA-co-TFEMA) terpolymers were prepared by radical polymerization of the methacrylic monomer derived from TIBA (M3I) with HEMA and TFEMA in solution and at high conversion. Previously, the HEMA was purified according to the standard procedure described in the literature (European Polymer Journal, 1976, 12, 685-9).

To that end, the M3I, HEMA and TFEMA monomers were weighed and dissolved in a Pyrex container having a capacity suitable for carrying out polymerization.

The volume of anhydrous DMSO was then added and it was necessary to wait for complete dissolution of the comonomers of the reaction. The necessary amount of AIBN was subsequently added as a polymerization initiator.

The reaction mixture was deoxygenated under nitrogen (N₂) atmosphere for 5 minutes and maintained in an oven for 24 hours at 60 °C, without stirring. Once the polymerization time elapsed, the reaction mixture was cooled to stop polymerization. The solution was subsequently precipitated on water with vigorous stirring in an ice bath. The obtained precipitate was then washed with water 3 times to remove DMSO. Finally, the obtained product was isolated by lyophilization.

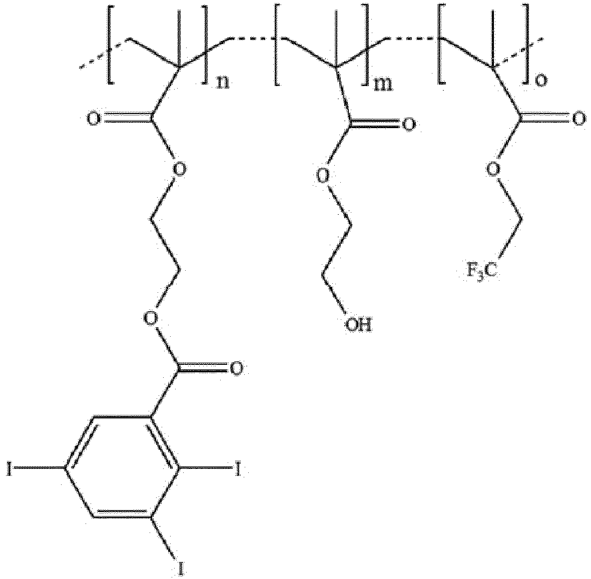

Following this methodology, different polymerization reactions were prepared by varying the molar composition in the comonomer feed, the molarity of the solution (M) and the concentration of AIBN.

### Iodinated terpolymer 2: poly(M3I-co-VP-co-TFEMA)

The poly(M3I-co-VP-co-TFEMA) terpolymers were prepared by radical polymerization of the methacrylic monomer derived from TIBA (M3I) with VP and TFEMA in solution and at high conversion. Previously, the VP was purified by vacuum distillation.

To that end, the M3I, VP and TFEMA monomers were weighed and dissolved in a Pyrex container having a capacity suitable for carrying out polymerization. The volume of anhydrous DMSO was then added and it was necessary to wait for complete dissolution of the comonomers of the reaction. The necessary amount of AlBN was subsequently added as a polymerization initiator.

The reaction mixture was deoxygenated under nitrogen (N₂) atmosphere for 5 minutes and maintained in an oven for 24 hours at 60 °C, without stirring. Once the polymerization time elapsed, the reaction mixture was cooled to stop polymerization. The solution was subsequently precipitated on water with vigorous stirring in an ice bath. The obtained precipitate was then washed with water 3 times to remove DMSO. Finally, the obtained product was isolated by lyophilization.

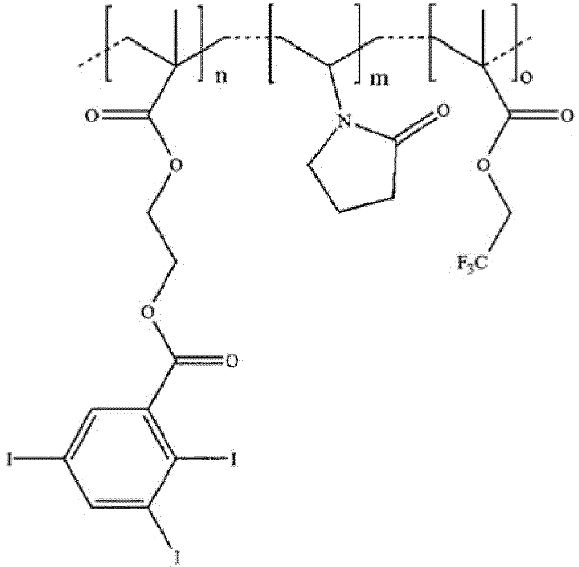

Following this methodology, different polymerization reactions were prepared by varying the molar composition in the comonomer feed, the molarity of the solution (M) and the concentration of AIBN.

### Brominated copolymer 1: poly(M5Br-co-HEMA)

The poly(M5Br-co-HEMA) copolymers were prepared by radical polymerization of the methacrylic monomer derived from pentabromophenol (M5Br) with HEMA in solution, at high conversion.

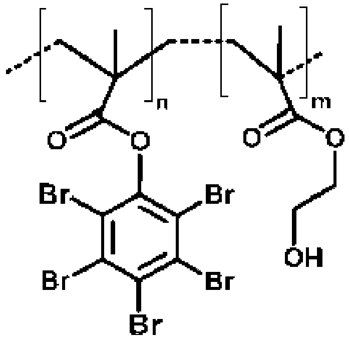

Previously, the HEMA was purified according to the standard procedure described in the literature (European Polymer Journal, 1976, 12, 685-9).

For carrying out polymerization, the comonomers were weighed and dissolved in a Pyrex container having a suitable capacity. The necessary volume of anhydrous DMSO was then added and it was necessary to wait for complete dissolution of the comonomers of the reaction. The necessary amount of AlBN was subsequently added as a polymerization initiator.

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath. The solid was then left to decant in the refrigerator for 2-3 h. Finally, the obtained precipitate was washed using a centrifuge (10000 rpm cycles for 5 min) with water 3 times to remove DMSO. Finally, the obtained product was isolated by lyophilization.

Following this methodology, different polymerization reactions were prepared by varying the molar composition in the comonomer feed, the molarity of the solution (M) and the concentration of AlBN.

### Brominated copolymer 2: poly(M5Br-co-VP)

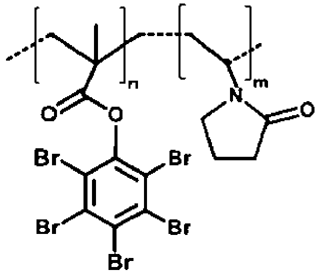

The poly(M5Br-co-VP) copolymers were prepared by radical polymerization of the methacrylic monomer derived from pentabromophenol (M5Br) with VP in solution, at high conversion. Previously, the VP was purified by vacuum distillation.

For carrying out polymerization, the comonomers were weighed and dissolved in a Pyrex container having a suitable capacity. The necessary volume of anhydrous DMSO was then added and it was necessary to wait for complete dissolution of the comonomers of the reaction. The necessary amount of AIBN was subsequently added as a polymerization initiator.

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath. The solid was then left to decant in the refrigerator for 2-3 h. Finally, the obtained precipitate was washed using a centrifuge (10000 rpm cycles for 5 min) with water 3 times to remove DMSO. Finally, the obtained product was isolated by lyophilization.

Following this methodology, different polymerization reactions were prepared by varying the molar composition in the comonomer feed, the molarity of the solution (M) and the concentration of AIBN.

### Brominated copolymer 3: poly(M5Brext-co-HEMA)

For carrying out polymerization, the M5Brext and HEMA comonomers were weighed and dissolved in a Pyrex container having a capacity suitable for carrying out polymerization. Previously, the HEMA was purified according to the standard procedure described in the literature (European Polymer Journal, 1976, 12, 685-9).

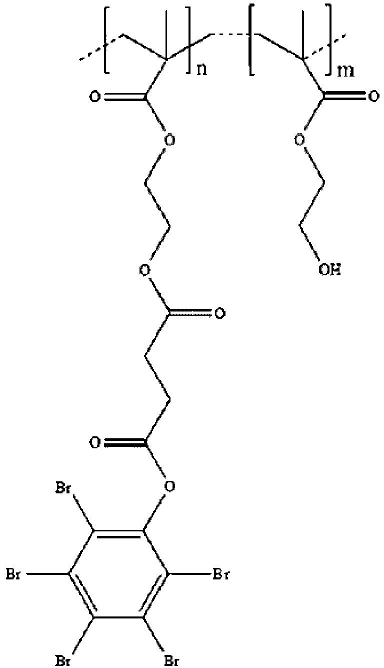

The necessary volume of anhydrous DMSO was then added and it was necessary to wait for complete dissolution of the comonomers of the reaction. The necessary amount of AlBN was subsequently added as a polymerization initiator.

The reaction mixture was deoxygenated under N₂ atmosphere for 5 minutes and maintained in an oven for 24 hours at 60 °C, without stirring. Once the polymerization time elapsed, the reaction mixture was cooled to stop polymerization. The solution was subsequently precipitated drop-wise on water (in a 2 l-capacity beaker with about 1.0-1.5 l of water) with vigorous stirring in an ice bath.

The solid was then left to decant in the refrigerator for 2-3 h. Finally, the obtained precipitate was washed using a centrifuge (10000 rpm cycles for 5 min) with water 3 times to remove DMSO. Finally, the obtained product was isolated by lyophilization.

Following this methodology, different polymerization reactions were prepared by varying the molar composition in the comonomer feed, the molarity of the solution (M) and the concentration of AIBN.

### Brominated copolymer 4: poly(M5Brext-co-VP)

For carrying out polymerization, the M5Brext and VP comonomers were weighed and dissolved in a Pyrex container having a capacity suitable for carrying out polymerization. Previously, the VP was purified by vacuum distillation.

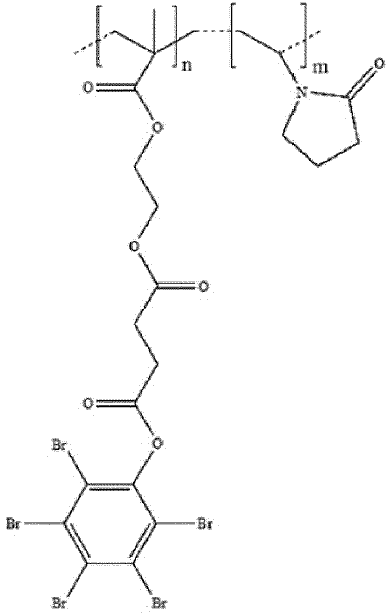

The necessary volume of anhydrous DMSO was then added and it was necessary to wait for complete dissolution of the comonomers of the reaction. The necessary amount of AIBN was subsequently added as a polymerization initiator.

The reaction mixture was deoxygenated under N₂ atmosphere for 5 minutes and maintained in an oven for 24 hours at 60 °C, without stirring. Once the polymerization time elapsed, the reaction mixture was cooled to stop polymerization.

The solution was subsequently precipitated drop-wise on water (in a 2 l-capacity beaker with about 1.0-1.5 l of water) with vigorous stirring in an ice bath. The solid was then left to decant in the refrigerator for 23 h. Finally, the obtained precipitate was washed using a centrifuge (10000 rpm cycles for 5 min) with water 3 times to remove DMSO. Finally, the obtained product was isolated by lyophilization.

Following this methodology, different polymerization reactions were prepared by varying the molar composition in the comonomer feed, the molarity of the solution (M) and the concentration of AIBN.

### Example of preparation of a liquid embolic agent

3.5 g of polymer are added to 10 g of DMSO. It is stirred to complete solubilization. A vial is filled or a syringe is pre-filled and they are autoclaved at 121 °C for 15 min.

### Example of application of the liquid embolic agent in an animal model

To conduct the experimental study, 25 domestic pigs were used in the application of a liquid embolic agent. The results were analyzed in 5 animals for an acute follow-up period (early; 24 hours) and in 10 animals for a sub-acute follow-up period (short-term; 1 month) and a chronic follow-up period (long-term; 3 months).

The efficacy and safety were evaluated for each follow-up period according to the criteria described below. Before being sacrificed, an angiographic assessment was carried out for each animal:

### a) Efficacy assessment:

Clinical success is defined as the correct embolization of the region to be devascularized. To that end, the penetration, distribution and durability characteristics of the embolization in the different assay periods, as well as the capacity for occlusion of the vessel and recanalization thereof, are analyzed by means of angiography and a histological study.

### b) Safety assessment:

By means of angiography: Vasospasm and tissue devascularization were studied in the acute phase. Revascularization was studied in the sub-acute and chronic phase.

By means of histological analysis: Vascular damage and the inflammatory reaction, hemorrhage, extravasation of the neovascularization agent were assessed in all phases.

The devices used were:
- Liquid embolic agent according to the invention
- Liquid embolic control Onyx-18 of eV3

In each animal, the following were assessed:
- Rete Mirabile: of the two Retes, one was embolized and the other one was as control (using contrast).
- Kidney: 1/3 of the kidney branch of the inferior renal polar artery is embolized. The two inferior poles of both kidneys in each animal are embolized.

After the scheduled follow-up period, a control angiography of the treated organs was performed before euthanizing the animals.

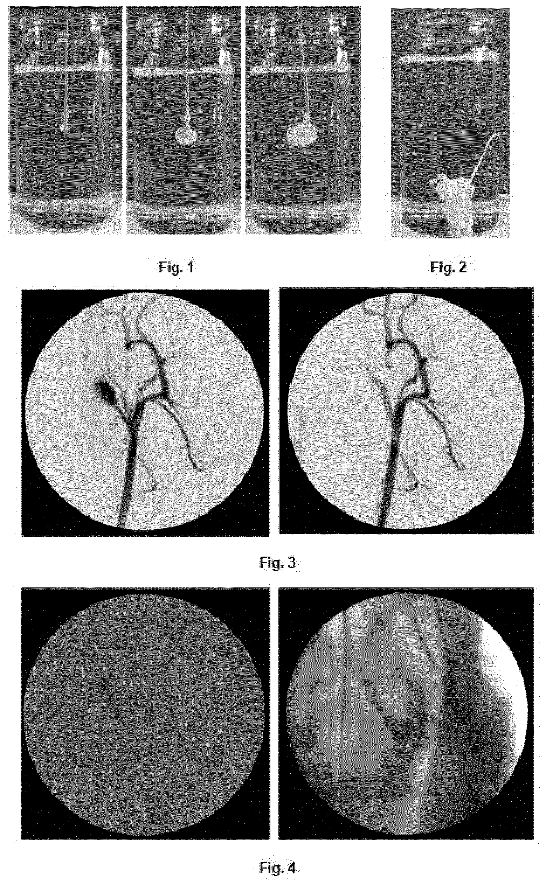

The result of the embolization procedure can be seen in Figure 3. The angiographic image on the left shows the rete mirabile of a porcine animal model, with complete vascularization of the organ being observed. The angiographic image on the right shows the result of the embolization process, wherein complete occlusion of the artery and the nidus of the rete mirabile has been achieved. The stained part corresponds to the non-embolized region.

Based on the study, it is concluded that the product has good technical features. The liquid embolic agent allows complete control from the injection, assessing the advance of the material until it stops when it is not injected. The reflux observed in all cases was less than 3 cm from the tip of the microcatheter. At no time was entrapment of the catheter observed at the time of removing it. This phenomenon can be observed with Onyx during the procedure, including entrapment of the catheter and risks of damage in the vessel due to the force needed to free it. This has led in practice to the design of a specific microcatheter with a detachable tip [Apollo (eV3; Irvine, CA)] for being used in these circumstances and for helping to prevent complications.

Visualization of the product is correct without the creation of artifacts in the control angiographies performed at the end of the study period. This result is highly valued for possible future interventions, unlike what occurs when working with Onyx.

It is concluded from the experiments performed that the liquid embolic agent is safe and effective for vascular embolizations, without observing angiotoxicity or systemic complications.

## Claims

1. A polymer of formula (I): 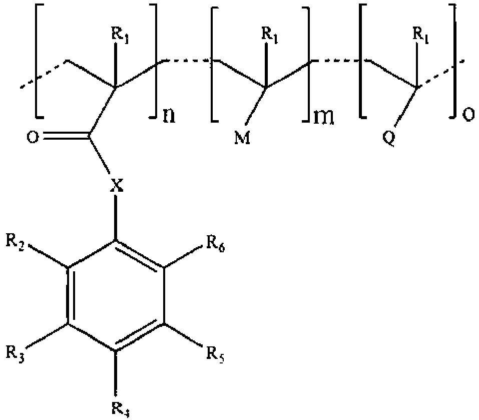 wherein:
n = m = o ≠ 0 terpolymer
n = m ≠ 0 and o =0 copolymer
n ≠ 0 and m = o = 0 homopolymer
R₁ = H, CH₃
R₂ = R₃ = R₄ = R₅ = R₆ = I, Br, H
**X=** -O-; CH₂-O-; 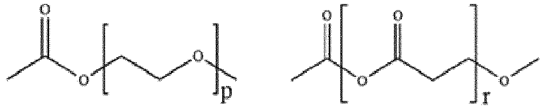 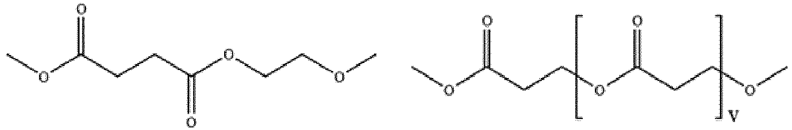 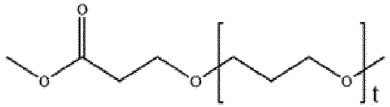
where p = 1, 6, r = 3, v = 3, and t = 6, **M=** 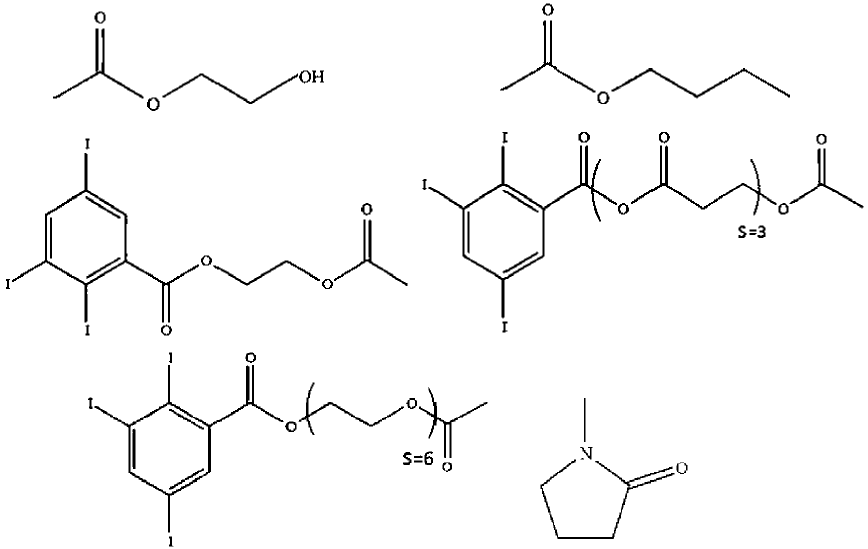
**Q=** 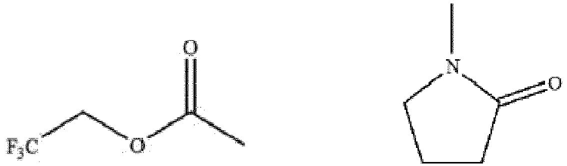
and wherein "n", "m" and "o" take values such that the molecular weight of the polymer is comprised between 50 kDa to 900 kDa.

2. A polymer according to claim 1, having a molecular weight comprised between 200 kDa to 800 kDa.

3. A polymer according to claim 1, having a proportion of the monomer unit of formula: 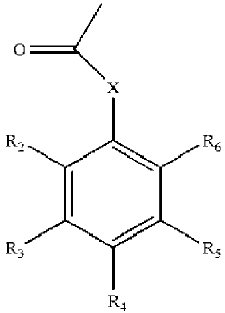 with respect to the remaining monomers of 40/60 to 100/0.

4. A polymer according to claim 1, having the formula selected from:

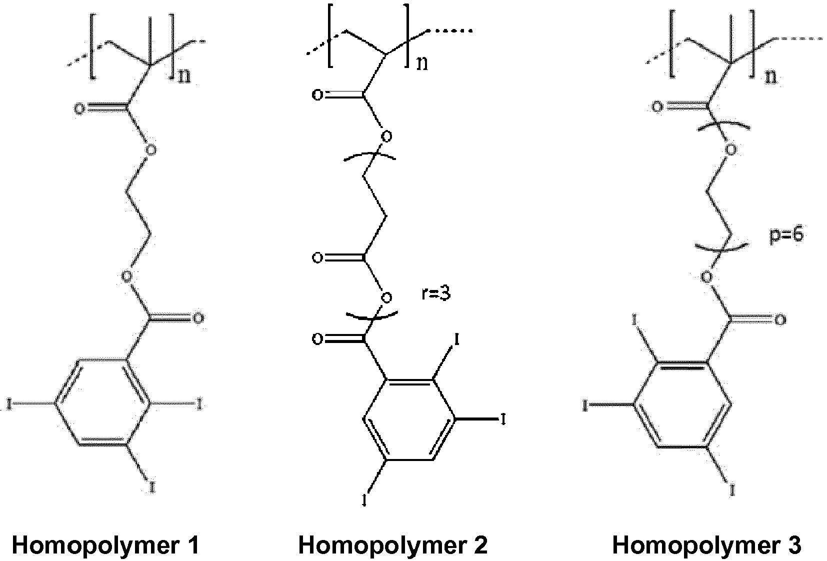

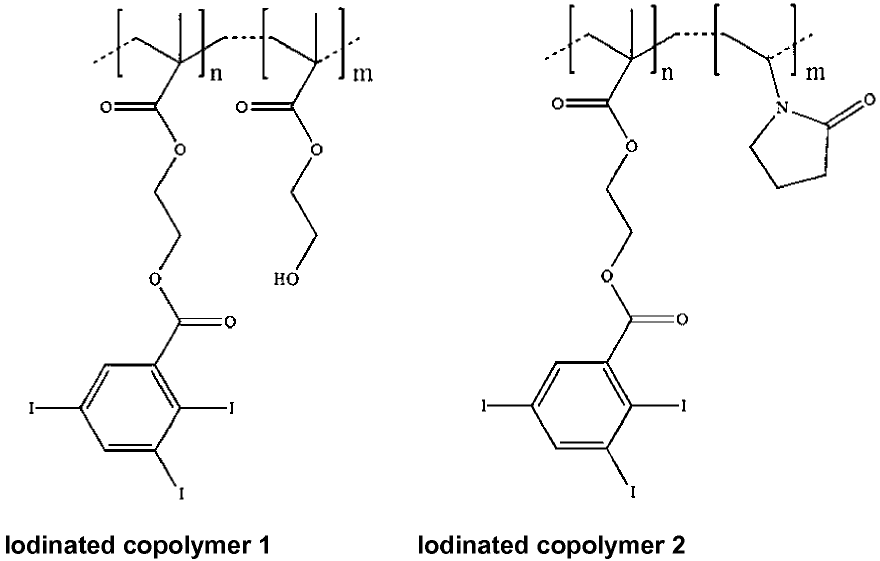

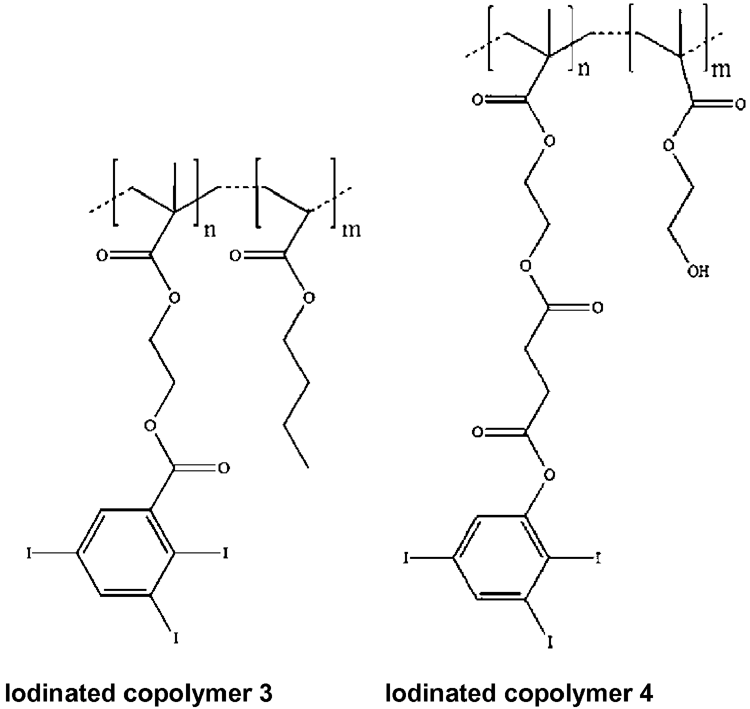

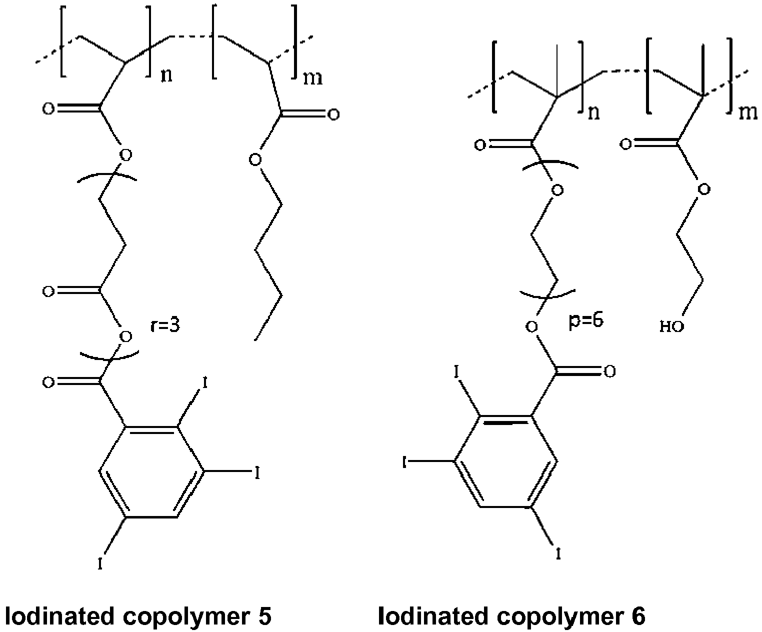

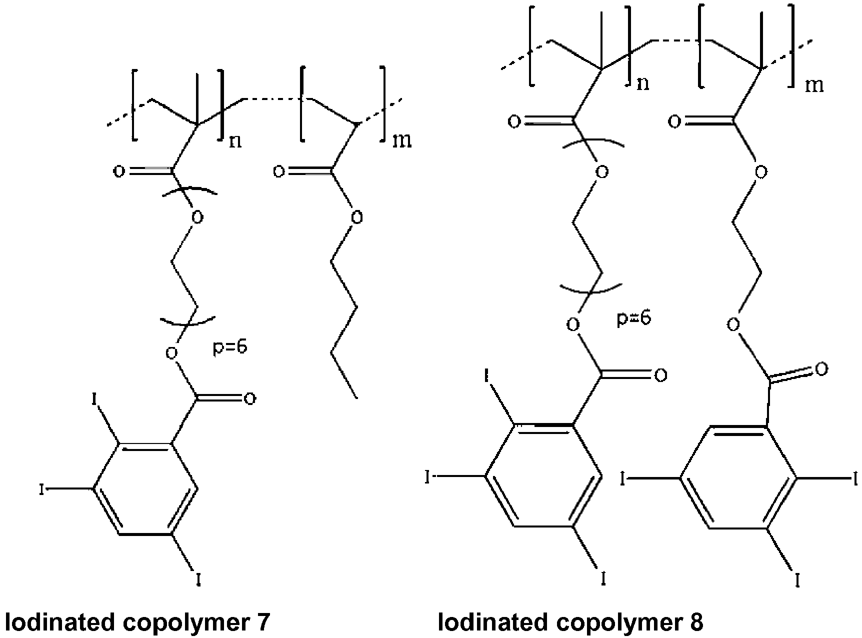

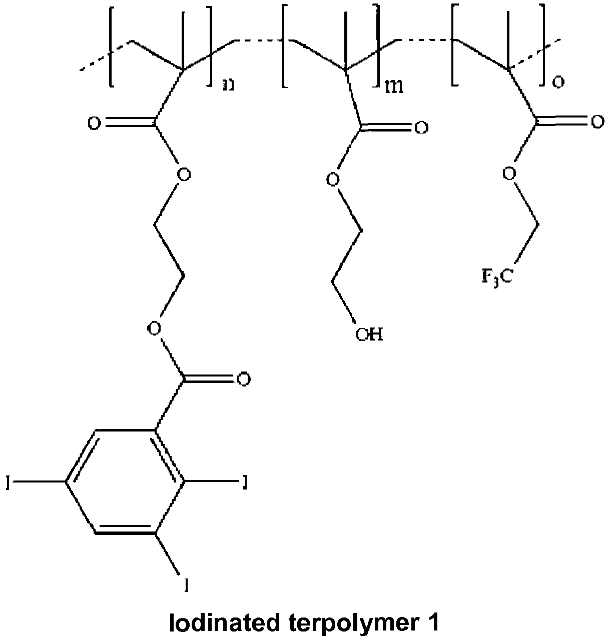

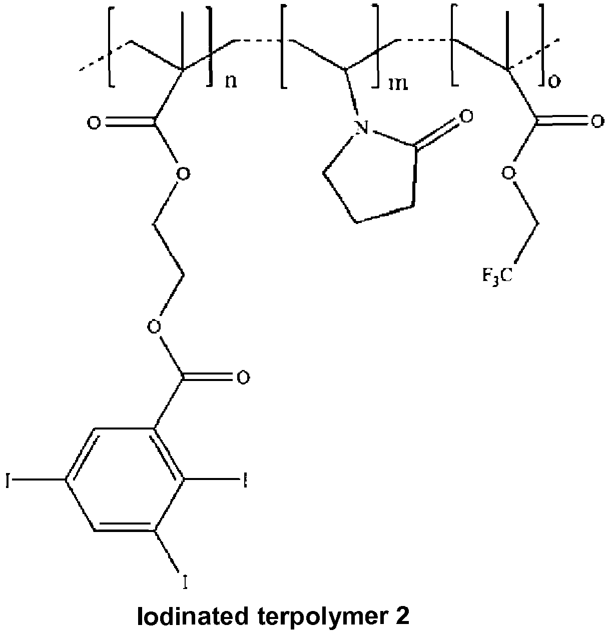

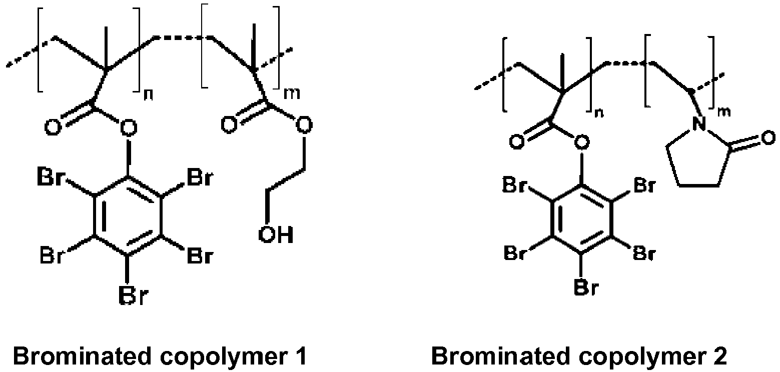

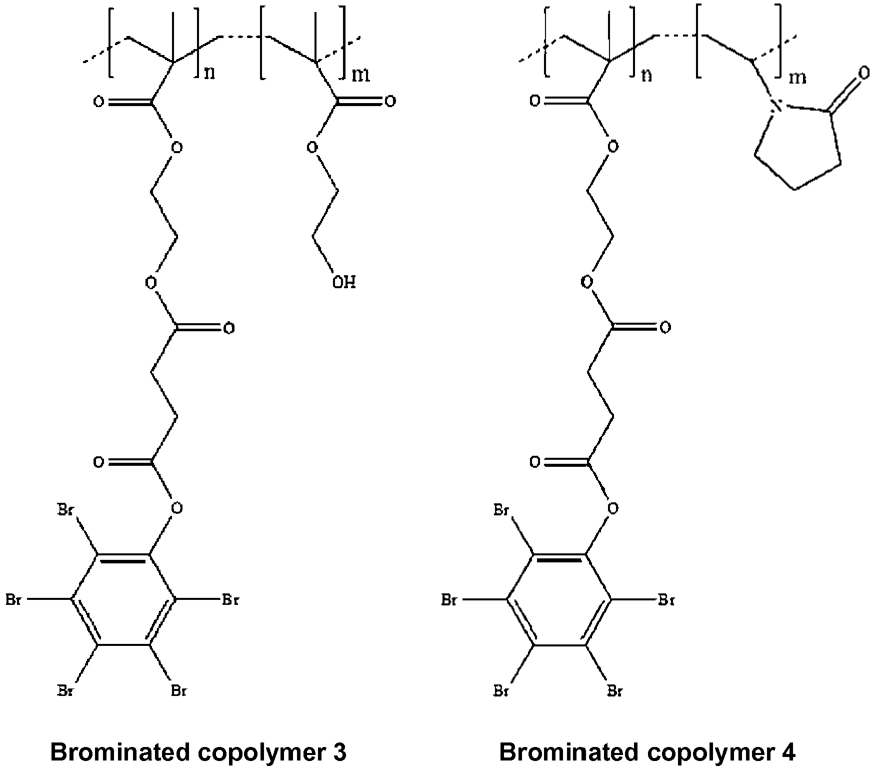

5. A monomer unit precursor of the polymer defined in claim 1, having formula II: 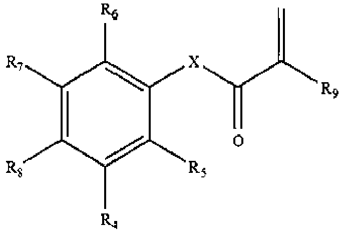 wherein:
R₄ = R₅ = R₆ = R₇ = R₈ = I, Br, H
R₉ can be H, CH3
**X=** -O-; CH₂-O-; 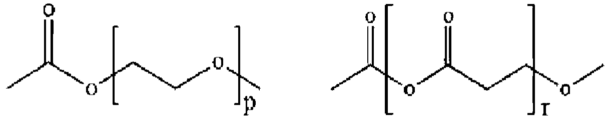 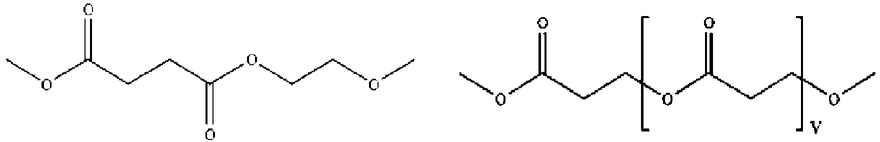 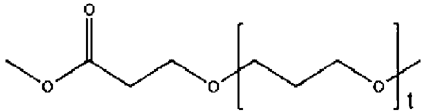
where p = 1, 6, r = 3, v = 3, and t = 6

6. A monomer unit according to claim 5, having the formula selected from:
**Iodinated monomer 1:** 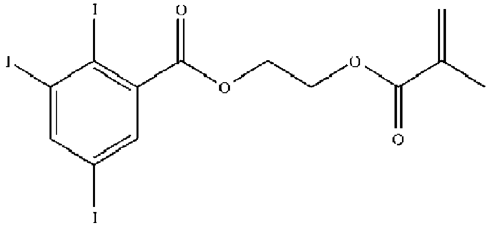
**Iodinated monomer 2:** 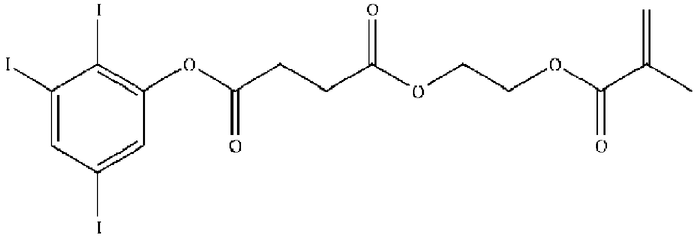
**Iodinated monomer 3:** 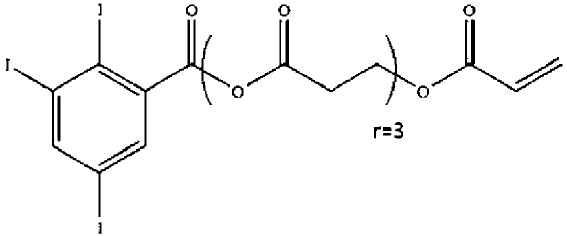
**Iodinated monomer 4:** 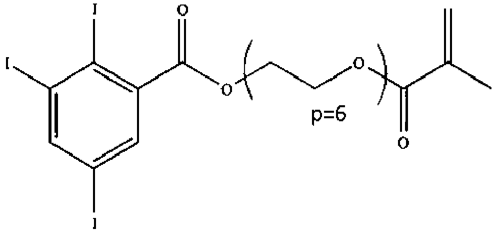
**Brominated monomer 1:** 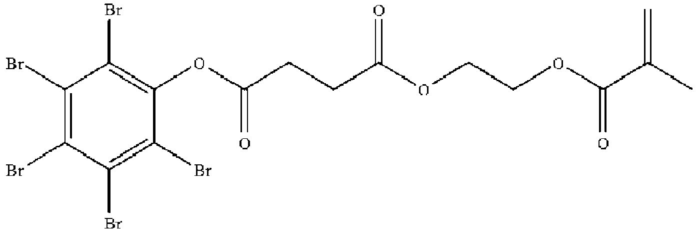
**Brominated monomer 2:** 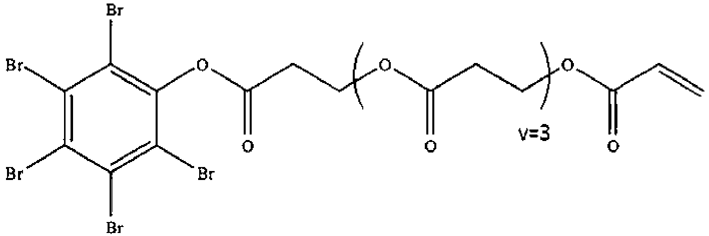
**Brominated monomer 3:**

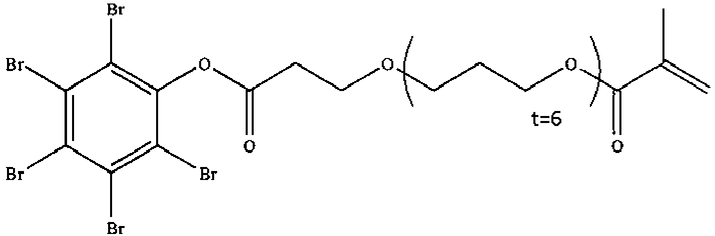

7. A method for the preparation of the previously defined polymers which comprises reacting a compound of formula II with one or two compounds selected from:
- the same monomer of formula (II)
- 2-hydroxyethyl methacrylate (HEMA)
- n-butyl acrylate (NBA)
- 1-vinyl-2-pyrrolidinone, (VP)
- 2,2,2-trifluoroethyl methacrylate (TFEMA).

8. A method according to claim 7, which method comprises carrying out a polymerization reaction: preferably in a water-miscible organic solvent; for 24 hours at a temperature comprised between 55 and 65 °C, preferably at 60 °C; at a molar concentration comprised between 0.2 and 0.5 M, more preferably between 0.3 and 0.35 M; with a concentration of thermal initiator AIBN comprised between 0.01 and 0.03 M, preferably 0.015 M.

9. A method according to claim 8, which method comprises carrying out a polymerization reaction: preferably in a water-miscible organic solvent; for 24 hours at a temperature of 60 °C; at a molar concentration comprised between 0.3 and 0.35 M; with a concentration of thermal initiator AIBN of 0.015 M.

10. A liquid embolic agent comprising a radiopaque polymer *per se,* as defined in claim 1, solubilized in a water-miscible organic solvent.

11. A liquid embolic agent according to claim 10, wherein the concentration of the biocompatible polymer/copolymer in the water-miscible organic solvent is 10 % to 60 %.

12. Use of the liquid embolic agent defined in claim 10 or 11 for vascular embolization.
